# EUROPEAN PATENT SPECIFICATION

(11) **EP 2 868 325 B1**
(45) Date of publication and mention of the grant of the patent: **01.11.2017**
(21) Application number: 13813675.9
(22) Date of filing: 02.07.2013
(51) Int. Cl.: A61K 38/00, A61K 9/06, A61K 9/08, A61K 9/107, A61K 9/70, A61K 47/10, A61K 47/12, A61K 47/32, A61P 35/00, A61P 43/00, C07K 7/06

(54) **TRANSDERMAL CANCER ANTIGEN PEPTIDE PREPARATION**
TRANSDERMALES KREBS-ANTIGEN-PEPTIDPRÄPARAT
PRÉPARATION TRANSDERMIQUE DE PEPTIDE ANTIGÉNIQUE ASSOCIÉ AU CANCER

(30) Priority: 02.07.2012 JP 2012148639
(43) Date of publication of application: 06.05.2015
(73) Proprietor: Sumitomo Dainippon Pharma Co., Ltd., Osaka-shi Osaka 541-8524 (JP)
(72) Inventor: TANAKA, Masayasu, Ibaraki-shi Osaka 5670878 (JP); YAMAMOTO, Kazumitsu, Ibaraki-shi Osaka 5670878 (JP); MAEDA, Hiroo, Ibaraki-shi Osaka 5670878 (JP); SAITO, Koichi, Ibaraki-shi Osaka 5670878 (JP); SUGINOBE, Natsuko, Osaka-shi Osaka 5540022 (JP)
(74) Representative: J A Kemp
(86) International application number: PCT/JP2013/068182
(87) International publication number: WO 2014/007266

(56) References cited:
- EP-A1- 2 762 152
- EP-A1- 2 762 159
- EP-A1- 2 974 734
- WO-A1-2006/118246
- WO-A1-2008/108257
- WO-A1-2009/066462
- WO-A1-2009/072610
- WO-A1-2010/103845
- JP-A- 2000 212 073
- JP-A- 2011 030 981
- JP-A- 2012 501 347

## Description

### Technical Field

The present invention belongs to the field of cancer immunotherapy, and relates to a transdermal preparation for WT1 protein-derived cancer antigen peptide having a cytotoxic T cell induction activity, and to a transdermal preparation containing a particular ether-type additive.

### Background Art

The immune mechanism for elimination of foreign substances is generally divided into liquid immunity and cellular immunity. In the liquid immunity, macrophage that recognizes antigen and functions as an antigen presenting cell, helper T cell that recognizes presentation of antigen of the macrophage, produces various lymphokine and activates other T cell and the like, and B lymphocyte that differentiates into antibody producing cell by the action of the lymphokine and the like are involved. In the cellular immunity, cytotoxic T cells after differentiation by the presentation of antigen attack and destroy target cells.

For elimination of cancer cells, virus infected cells and the like in the body, cellular immunity, particularly cytotoxic T cell (cytotoxic T-lymphocyte, Cytotoxic T-cell, hereinafter to be referred to as CTL) mainly plays an important role. CTL is produced by differentiation and proliferation of a precursor T cell that recognized a complex formed by an antigen peptide derived from a cancer antigen protein (cancer antigen peptide) and Major Histocompatibility Complex (MHC) class I antigen, and the thus-produced CTL attacks and destroys cancer cells. In this event, cancer cells present a complex of MHC class I antigen and cancer antigen on the cellular surface, and the complex becomes the target of CTL (see non-patent documents 1-4). MHC in human is called human leukocyte antigen (HLA).

For a cancer immunotherapeutic agent utilizing the destruction of cancer cells by CTL, that is, cancer vaccine, the development as a preparation that efficiency induces CTL is desired.

The cancer antigen that binds to MHC class I antigen and is presented on cancer cells to be the target cells is a peptide produced by degradation (processing) of cancer antigen protein, synthesized in cancer cells, by intracellular protease, which is considered to be a peptide consisting of 8 - 12 amino acid residues (see non-patent documents 1-4).

plural cancer antigen peptides have been reported as follows relating to, among many cancer antigen proteins, a protein which is a gene product of cancer suppressor gene WT1 of Wilms tumor, i.e., WT1 protein (SEQ ID NO: 1).

Wilms tumor gene WT1 is isolated as a gene involved in the formation of Wilms tumor, which is childhood kidney tumor (see non-patent document 5). This gene encodes a regulating mechanism for cell proliferation and cell differentiation, as well as a zinc finger transcription factor related to apoptosis and tissue development.

WT1 gene was first classified as a tumor suppressor gene; however, it is suggested in recent years that wild-type WT1 gene performs oncogenic action rather than tumor suppressive action in various types of malignant diseases, based on the following evidence (i) - (iii);
(i) high expression of wild-type WT1 gene in various human malignant tumors and solid tumors including hematopoietic organ malignant tumors such as leukemia and myelodysplastic syndrome (MDS),
(ii) inhibition of growth of human leukemic cell and solid tumor cell treated with WT1 antisense or oligonucleotide,
(iii) promotion of the growth of and prevention of the differentiation of mouse myeloid progenitor cell due to constitutional expression of wild-type WT1 gene (see non-patent document 6).

Furthermore, WT1 gene is also known to highly express in solid tumors such as gastric cancer, colorectal cancer, lung cancer, breast cancer, germ cell cancer, liver cancer, skin cancer, urinary bladder cancer, prostate cancer, uterine cancer, cervical cancer, ovarian cancer and the like (see non-patent document 7).

As cancer antigen peptide of such WT1 gene, namely, human WT1 protein-derived cancer antigen peptide, WT1₁₂₆₋₁₃₄ peptide (Arg-Met-Phe-Pro-Asn-Ala-Pro-Tyr-Leu, i.e., RMFPNAPYL (SEQ ID NO: 2)), WT1₁₈₇₋₁₉₅ peptide (Ser-Leu-Gly-Glu-Gln-Gln-Tyr-Ser-Val, i.e., SLGEQQYSV (SEQ ID NO: 3)), WT1₂₃₅₋₂₄₃ peptide (Cys-Met-Thr-Trp-Asn-Gln-Met-Asn-Leu, i.e., CMTWNQMNL (SEQ ID NO: 6)), WT1₁₀₋₁₈ peptide (Ala-Leu-Leu-Pro-Ala-Val-Pro-Ser-Leu, i.e., ALLPAVPSL (SEQ ID NO: 5)) and the like have been reported (see patent document 1, patent document 2). While these peptides are merely one embodiment of WT1 protein-derived cancer antigen peptide, it is evident that the amino acid composition varies among these peptides in one embodiment.

The property of cancer antigen peptide varies depending on the amino acid composition. As for water solubility, for example, some cancer antigen peptides show high water solubility, whereas some show poor solubility. Therefore, a preparation having broad utility and capable of efficient CTL induction for various cancer antigen peptides, without being influenced by the kind and property of the cancer antigen peptides is desirable.

As a cancer vaccine preparation developed heretofore, preparations that can be administered by injection accompanying invasiveness such as intradermal administration, subcutaneous administration, intramuscular administration and the like are general. On the other hand, transdermal administration is useful since it does not accompany invasiveness, can maintain drug concentration in the vicinity of the administration site in a more sustained manner, and can lower liver metabolism and drug interaction by avoiding first pass effect. In addition, administration by way of a transdermal preparation, particularly patches preparation, has many superior points such as easy confirmation and discontinuation of medication, absence of influence of diet, and the like.

However, since skin functions as a barrier to prevent invasion of foreign substances from the outside, it is difficult to deliver, into the body, a drug in an amount necessary and sufficient to provide efficacy by simply applying or attaching the drug to the skin. Generally, therefore, a method of expressing the efficacy by blending an additive aiming at promotion of skin permeation of the drug, namely, a transdermal absorption promoter, in a preparation and administering the drug is adopted. As an example of immunostimulation promoter in cancer immunotherapy, it is known that a low molecular weight carboxylic acid compound such as L-lactic acid and the like promote immunostimulation by cancer antigen and the like (see patent document 3). As an example of a transdermal absorption promoter, for example, terpenes such as limonene and the like are known (see patent document 10). Under such background, the development of a transdermal absorption promoter which is safe, superior in usability, and shows high effect, irrespective of the kind of the drug, has been desired.

WO 2008/108257 and WO 2009/066462 disclose WT1 peptides for inducing CTL.

WO 2006/118246 and WO 2010/103845 disclose ethers having a promoting effect on the transdermal absorption of specific peptides and proteins.

JP 2000 212073 discloses a transdermal patch with improved absorption.

### [Document List]

### [patent documents]

patent document 1: WO 00/06602
patent document 2: WO 00/18795
patent document 3: JP-A-2008-127277
patent document 4: WO 02/079253
patent document 5: WO 03/106682
patent document 6: WO 2004/026897
patent document 7: WO 2009/072610
patent document 8: WO 2007/063903
patent document 9: WO 2004/063217
patent document 10: JP-A-3-63233

### [non-patent documents]

non-patent document 1: Current Opininon in Immunology, 1993; 5(5); 709-713
non-patent document 2: Current Opininon in Immunology, 1993; 5(5); 719-725
non-patent document 3: Cell, 1995; 82(1); 13-17
non-patent document 4: Immunological Reviews, 1995; 146(1); 177-210
non-patent document 5: The FASEB journal, 1993; 7; 896-903
non-patent document 6: International Journal of Hematology, 2001; 73(2); 177-187
non-patent document 7: Cancer Science, 2004; 95(7); 583-587

### SUMMARY OF THE INVENTION

### Problems to be Solved by the Invention

The problem of the present invention is to provide a transdermal preparation for WT1 protein-derived cancer antigen peptide capable of efficiently inducing CTL.

### Means of Solving the Problems

The present inventors have conducted intensive studies in an attempt to solve the above-mentioned problem and found that a good CTL induction by WT1 protein-derived cancer antigen peptide can be achieved by adding a particular ether-type additive as an additive, which resulted in the completion of the present invention.

Accordingly, the present invention relates to the following.

Item 1. A transdermal preparation comprising a WT1 protein-derived cancer antigen peptide, and an ether-type additive represented by the formula (1):

R¹-Q-R² (1)

[wherein R¹ is a hydrocarbon group having 8 - 24 carbon atoms, and
R² is a group represented by the formula (2): 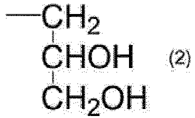
or a group represented by the formula (3):

-(CH₂CH₂O)ₘH (3)

wherein m is an integer of 1 - 18],
wherein the additive is liquid at 20°C,
further comprising lactic acid, and
further comprising at least one kind of WT1 protein-derived cancer antigen peptide degradation inhibitor selected from the group consisting of decanoic acid, sodium deoxycholate or N-lauroylsarcosine.

Item 2. The transdermal preparation according to item 1, wherein the WT1 protein-derived cancer antigen peptide is a peptide comprising any amino acid sequence selected from the following amino acid sequences:
Arg-Met-Phe-Pro-Asn-Ala-Pro-Tyr-Leu (SEQ ID NO: 2),
Ser-Leu-Gly-Glu-Gln-Gln-Tyr-Ser-Val (SEQ ID NO: 3),
Cys-Tyr-Thr-Trp-Asn-Gln-Met-Asn-Leu (SEQ ID NO: 4), and
Ala-Leu-Leu-Pro-Ala-Val-Pro-Ser-Leu (SEQ ID NO: 5) or
a peptide comprising an altered amino acid sequence, which is any amino acid sequence selected from SEQ ID NOs: 2, 3, 4 and 5 but containing alteration of amino acid residue(s), and having a CTL induction activity.

Item 3. The transdermal preparation according to item 1 or 2, wherein the WT1 protein-derived cancer antigen peptide is a peptide shown in the amino acid sequence
Arg-Met-Phe-Pro-Asn-Ala-Pro-Tyr-Leu (SEQ ID NO: 2).

Item 4. The transdermal preparation according to any one of items 1 - 3, wherein the ether-type additive is represented by the formula (4): 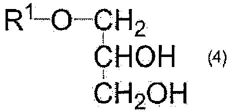 [wherein R¹ is a hydrocarbon group having 8 - 24 carbon atoms, or the formula (5):

R¹-O-(CH₂CH₂O)ₙH (5)

wherein R¹ is a hydrocarbon group having 8 - 24 carbon atoms, and
n is an integer of 2 - 18].

Item 5. The transdermal preparation according to any one of items 1 - 4, wherein the ether-type additive is represented by the formula (6): 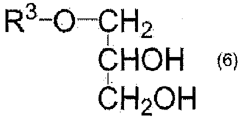 [wherein R³ is a hydrocarbon group having 16 - 18 carbon atoms].

Item 6. The transdermal preparation according to any one of items 1 - 4, wherein the ether-type additive is represented by the formula (7):

R¹-O-(CH₂CH₂O)ₚH (7)

[wherein R¹ is a hydrocarbon group having 8 - 24 carbon atoms, and
p is an integer of 2 - 12].

Item 7. The transdermal preparation according to item 6, wherein R¹ is a branched alkyl group having 8 - 24 carbon atoms, a linear alkenyl group having 8 - 24 carbon atoms or a mixed alkyl group having 8 - 24 carbon atoms.

Item 8. The transdermal preparation according to any one of items 1 - 4, wherein the ether-type additive is α-monoisostearyl glyceryl ether, monooleyl glyceryl ether, polyoxyethylene isostearyl ether which is liquid at 20°C, polyoxyethylene oleyl ether which is liquid at 20°C, polyoxyethylene alkyl (12 - 14) ether which is liquid at 20°C or a mixture of these.

Item 9. The transdermal preparation according to item 8, wherein the ether-type additive is α-monoisostearyl glyceryl ether and/or monooleyl glyceryl ether.

Item 10. The transdermal preparation according to any one of items 1 - 9, which has a dosage form of patches preparation, ointments, gels, creams, lotions or microneedle preparation.

Item 11. The transdermal preparation according to item 10, wherein the dosage form is patches preparation.

Item 12. The transdermal preparation according to item 8, which is a patches preparation comprising a support and an adhesive layer formed on one surface of the support, wherein the adhesive layer comprises (1) WT1 protein-derived cancer antigen peptide, (2) at least one ether-type additive selected from the group consisting of α-monoisostearyl glyceryl ether, monooleyl glyceryl ether, polyoxyethylene isostearyl ether which is liquid at 20°C, polyoxyethylene oleyl ether which is liquid at 20°C and polyoxyethylene alkyl (12 - 14) ether which is liquid at 20°C, and (3) an adhesive.

Item 13. The transdermal preparation according to item 12, wherein the adhesive is at least one kind selected from an acrylic adhesive, a rubber adhesive and a silicone adhesive.

Item 14. The transdermal preparation according to item 12 or 13, wherein the adhesive comprises an acrylic adhesive.

Item 15. The transdermal preparation according to any one of items 12 - 14, wherein the acrylic adhesive is at least one kind selected from the group consisting of a (co)polymer mainly comprising (meth)acrylic acid alkyl ester, and a copolymer of (meth)acrylic acid alkyl ester and a functional monomer.

Item 16. The transdermal preparation according to item 12 or 13, wherein the adhesive comprises a rubber adhesive.

Item 17. The transdermal preparation according to item 16, wherein the rubber adhesive is at least one kind selected from the group consisting of a styrene-isoprene-styrene block copolymer and polyisobutylene.

Item 18. A CTL inducer comprising a WT1 protein-derived cancer antigen peptide, and an ether-type additive represented by the formula (1) :

R¹-O-R² (1)

[wherein R¹ is a hydrocarbon group having 8 - 24 carbon atoms, and
R² is a group represented by the formula (2): 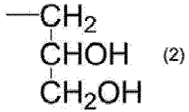
or a group represented by the formula (3):

-(CH₂CH₂O)ₘH (3)

wherein m is an integer of 1 - 18],
wherein the additive is liquid at 20°C,
further comprising lactic acid, and
further comprising at least one kind of WT1 protein-derived cancer antigen peptide degradation inhibitor selected from the group consisting of decanoic acid, sodium deoxycholate or N-lauroylsarcosine.

Item 19. The transdermal preparation according to item 11, wherein the patches preparation comprises an adhesive additive layer and an adhesive layer, wherein the adhesive additive layer comprises an ether-type additive represented by the formula (1) :

R¹-O-R² (1)

[wherein R¹ is a hydrocarbon group having 8 - 24 carbon atoms, and
R² is a group represented by the formula (2): 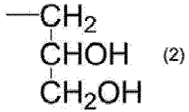
or a group represented by the formula (3):

-(CH₂CH₂O)ₘH (3)

wherein m is an integer of 1 - 18],
wherein the additive is liquid at 20°C, and the preparation does not substantially comprise a WT1 protein-derived cancer antigen peptide.

Item 20. The transdermal preparation according to item 19, wherein the adhesive layer comprises a WT1 protein-derived cancer antigen peptide, and an ether-type additive represented by the formula (1) :

R¹-O-R² (1)

[wherein R¹ is a hydrocarbon group having 8 - 24 carbon atoms, and
R² is a group represented by the formula (2): 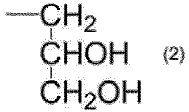
or a group represented by the formula (3):

-CH₂CH₂O)ₘH (3)

wherein m is an integer of 1 - 18],
wherein the additive is liquid at 20°C.

Item 21. The transdermal preparation according to item 20, wherein the same ether-type additive is contained in the adhesive layer and the adhesive additive layer.

Item 22. The transdermal preparation according to item 21, wherein the amount of the ether-type additives in the adhesive layer and the adhesive additive layer is the same.

Item 23. The transdermal preparation according to any one of items 19 - 22, wherein the adhesive layer and the adhesive additive layer are formed on the support.

Item 24. A method of producing the transdermal preparation according to any one of items 19 - 23, comprising a step of forming an adhesive additive layer on one surface of a support, a step of forming an adhesive layer on one surface of a release liner, and a step of adhering the surface of the adhesive additive layer of the support to the surface of the adhesive layer of the release liner.

### Effect of the Invention

As conventional preparations of cancer vaccine such as cancer antigen peptide and the like, injection preparations have been generally used. However, the present invention has enabled supply of a transdermal preparation containing an ether-type additive represented by the aforementioned formula (1) and liquid at 20°C, and also enabled transdermal administration of cancer antigen peptide without invasiveness due to the injection preparation. Since the transdermal administration has been enabled, the drug concentration can be maintained in the vicinity of the administration site for a longer time in a sustained manner. In addition, since first pass effect is avoided by the transdermal administration, metabolism in the liver and drug interaction are also expected to be reduced. Furthermore, transdermal administration facilitates confirmation and discontinuation of medication.

### Brief Description of the Drawings

Fig. 1 shows the test results of specific CTL induction in Experimental Example 1 for the tapes preparation 1 produced in Example 1, which contains the peptide shown in SEQ ID NO:2 and α-monoisostearyl glyceryl ether.
Fig. 2 shows the test results of specific CTL induction in Experimental Example 2 for the tapes preparation 2 produced in Example 2, which contains the peptide shown in SEQ ID NO:2 and monooleyl glyceryl ether.
Fig. 3 shows the test results of specific CTL induction in Experimental Example 2 for the tapes preparation 3 produced in Example 3, which contains the peptide shown in SEQ ID NO:2 and polyoxyethylene isostearyl ether.
Fig. 4 shows the test results of specific CTL induction in Experimental Example 2 for the tapes preparation 4 produced in Example 4, which contains the peptide shown in SEQ ID NO:2 and polyoxyethylene oleyl ether.
Fig. 5 shows the test results of specific CTL induction in Experimental Example 2 for the tapes preparation 5 produced in Example 5, which contains the peptide shown in SEQ ID NO:2 and polyoxyethylene alkyl (12 - 14) ether.
Fig. 6 shows the test results of specific CTL induction in Experimental Example 2 for the tapes preparation A produced in Reference Example 1, which contains the peptide shown in SEQ ID NO:2 and lactic acid.
Fig. 7 shows the test results of specific CTL induction in Experimental Example 2 for the tapes preparation B produced in Reference Example 2, which contains the peptide shown in SEQ ID NO:2 and isostearyl glyceryl ester.
Fig. 8 shows the test results of specific CTL induction in Experimental Example 2 for the tapes preparation C produced in Reference Example 3, which contains the peptide shown in SEQ ID NO:2 and isostearyl alcohol.
Fig. 9 shows the test results of specific CTL induction in Experimental Example 2 for the tapes preparation D produced in Reference Example 4, which contains the peptide shown in SEQ ID NO:2 and oleyl glyceryl ester.
Fig. 10 shows the test results of specific CTL induction in Experimental Example 2 for the tapes preparation E produced in Reference Example 5, which contains the peptide shown in SEQ ID NO:2 and polyoxyethylene polyoxypropylene cetyl ether.
Fig. 11 shows the test results of specific CTL induction in Experimental Example 3 for the tapes preparation 6 produced in Example 6, which contains the peptide shown in SEQ ID NO:2, α-monoisostearyl glyceryl ether, lactic acid and N-lauroylsarcosine. For comparison, the test results of specific CTL induction for the tapes preparation 1 produced in Example 1, which contains the peptide shown in SEQ ID NO:2 and α-monoisostearyl glyceryl ether are concurrently shown.

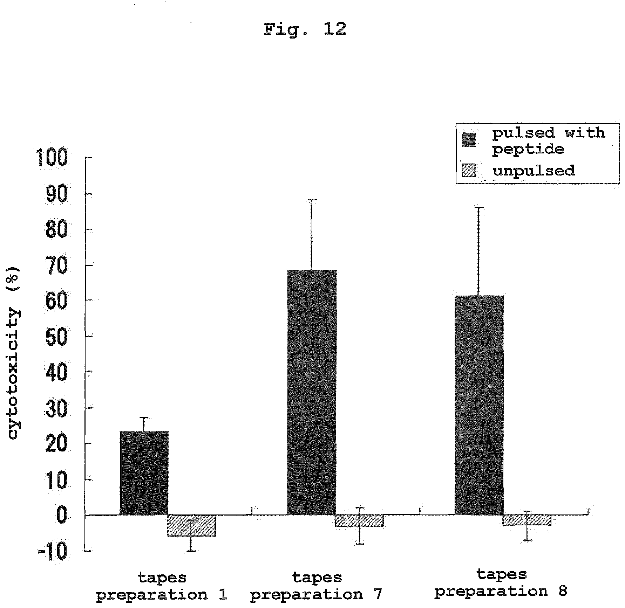

Fig. 12 shows the test results of specific CTL induction in Experimental Example 3 for the tapes preparation 7 produced in Example 7, which contains the peptide shown in SEQ ID NO:2, α-monoisostearyl glyceryl ether, lactic acid and sodium deoxycholate, and the tapes preparation 8 produced in Example 8, which contains the peptide shown in SEQ ID NO:2, α-monoisostearyl glyceryl ether, lactic acid and decanoic acid. For comparison, the test results of specific CTL induction for the tapes preparation 1 produced in Example 1, which contains the peptide shown in SEQ ID NO:2 and α-monoisostearyl glyceryl ether are concurrently shown.
Fig. 13 shows the test results of specific CTL induction in Experimental Example 4 for the tapes preparation 9 produced in Example 9, which contains the peptide shown in SEQ ID NO:2 and α-monoisostearyl glyceryl ether at a content percentage of 5%.
Fig. 14 shows the test results of specific CTL induction in Experimental Example 4 for the tapes preparation 10 produced in Example 10, which contains the peptide shown in SEQ ID NO:2, α-monoisostearyl glyceryl ether at a content percentage of 5% and polyoxyethylene isostearyl ether at a content percentage of 5%.
Fig. 15 shows the test results of specific CTL induction in Experimental Example 5 for the tapes preparation 11 produced in Example 11, which contains the peptide shown in SEQ ID NO:2 at a content percentage of 1%, α-monoisostearyl glyceryl ether, lactic acid and N-lauroylsarcosine.
Fig. 16 shows the test results of specific CTL induction in Experimental Example 6 for the tapes preparation 12 produced in Example 12, which contains the peptide shown in SEQ ID NO:2, α-monoisostearyl glyceryl ether at a content percentage of 1%, lactic acid and decanoic acid.
Fig. 17 shows the test results of specific CTL induction in Experimental Example 6 for the tapes preparation 13 produced in Example 13, which contains the peptide shown in SEQ ID NO:2, α-monoisostearyl glyceryl ether at a content percentage of 15%, lactic acid and decanoic acid.
Fig. 18 shows the test results of specific CTL induction in Experimental Example 7 for the tapes preparation 14 produced in Example 14, which contains the peptide shown in SEQ ID NO:2, polyoxyethylene isostearyl ether, lactic acid and decanoic acid.
Fig. 19 shows the test results of specific CTL induction in Experimental Example 7 for the tapes preparation 15 produced in Example 15, which contains the peptide shown in SEQ ID NO:2, polyoxyethylene 2-ethylhexyl ether, lactic acid and decanoic acid.
Fig. 20 shows the test results of specific CTL induction in Experimental Example 8 for the tapes preparation 16 produced in Example 16, which contains the peptide shown in SEQ ID NO:2 and α-monoisostearyl glyceryl ether.
Fig. 21 shows the test results of specific CTL induction in Experimental Example 8 for the tapes preparation 17 produced in Example 17, which contains the peptide shown in SEQ ID NO:2, α-monoisostearyl glyceryl ether, lactic acid and N-lauroylsarcosine.
Fig. 22 shows the test results of specific CTL induction in Experimental Example 8 for the tapes preparation 18 produced in Example 18, which contains the peptide shown in SEQ ID NO:2, α-monoisostearyl glyceryl ether, lactic acid and decanoic acid.

### Description of Embodiments

Embodiments of the present invention are explained in detail in the following.

The "WT1 protein-derived cancer antigen peptide" in the present invention is a partial peptide derived from a protein which is a gene product of cancer suppressive gene WT1 of Wilms tumor, that is, WT1 protein (SEQ ID NO: 1). To be specific, it is a peptide of 8 - 12 amino acids or a dimer thereof, and contains a peptide which is presented to MHC class I antigen and antigen-recognized by CTL. Of the peptides having 8 - 12 amino acids, a peptide having 9 amino acids is preferable.

Specific examples of the amino acid sequence of the WT1 protein-derived cancer antigen peptide include the following. The cancer antigen peptides having the amino acid sequences recited below are known.
RMFPNAPYL (SEQ ID NO: 2),
SLGEQQYSV (SEQ ID NO: 3),
CMTWNQMNL (SEQ ID NO: 6),
ALLPAVPSL (SEQ ID NO: 5),
RWPSCQKKF (SEQ ID NO: 7) and the like.

In the present specification, the above-mentioned peptides have the N terminal on the left side, and each amino acid symbol shows the following amino acid residue.
Ala or A: alanine residue
Arg or R: arginine residue
Asn or N: asparagine residue
Asp or D: aspartic acid residue
Cys or C: cysteine residue
Gln or Q: glutamine residue
Glu or E: glutamic acid residue
Gly or G: glycine residue
His or H: histidine residue
Ile or I: isoleucine residue
Leu or L: leucine residue
Lys or K: lysine residue
Met or M: methionine residue
Phe or F: phenylalanine residue
Pro or P: proline residue
Ser or S: serine residue
Thr or T: threonine residue
Trp or W: tryptophan residue
Tyr or Y: tyrosine residue
Val or V: valine residue
Abu: 2-aminobutyric acid residue (to be also referred to as α-aminobutyric acid residue)
Orn: ornithine residue
Cit: citrulline residue

The transdermal preparation or CTL inducer of the present invention may contain not only one kind but also two or more kinds of the WT1 protein-derived cancer antigen peptides. These WT1 protein-derived cancer antigen peptides can be produced by solid phase synthesis process by Fmoc method and the like, or other known methods.

In addition, the WT1 protein-derived cancer antigen peptide in the present invention may be a partial peptide of a natural cancer antigen protein (natural form, natural peptide, natural partial peptide), or an altered form wherein a part of the amino acids of natural partial peptide is altered (altered peptide), as long as induction of therapeutically effective specific CTL for each cancer antigen is not prevented in a transdermal preparation or CTL inducer to be finally obtained. In the alteration, the amino acid to be inserted or substituted may be a non-natural amino acid other than the 20 kinds of amino acids encoded by the gene.

The "WT1 protein-derived partial peptide" in the present invention is a gene product of zinc finger transcription factor isolated as a causative gene of Wilms tumor, and is a peptide that can bind to an HLA molecule to be a target antigen of a malignant tumor. To be specific, it is a partial peptide consisting of continuous 8 - 12 amino acids in the amino acid sequence of human WT1 protein (SEQ ID NO: 1) consisting of 449 amino acids, which binds to HLA to induce CTL. As mentioned above, examples thereof include WT1₁₂₆₋₁₃₄ peptide (Arg-Met-Phe-Pro-Asn-Ala-Pro-Tyr-Leu, i.e., RMFPNAPYL (SEQ ID NO: 2)), WT1₁₈₇₋₁₉₅ peptide (Ser-Leu-Gly-Glu-Gln-Gln-Tyr-Ser-Val, i.e., SLGEQQYSV (SEQ ID NO: 3)), WT1₂₃₅₋₂₄₃ peptide (Cys-Met-Thr-Trp-Asn-Gln-Met-Asn-Leu, i.e., CMTWNQMNL (SEQ ID NO: 6)), WT1₁₀₋₁₈ peptide (Ala-Leu-Leu-Pro-Ala-Val-Pro-Ser-Leu, i.e., ALLPAVPSL (SEQ ID NO: 5)), WT1₄₁₇₋₄₂₅ peptide (Arg-Trp-Pro-Ser-Cys-Gln-Lys-Lys-Phe, i.e., RWPSCQKKF (SEQ ID NO: 7)) and the like (see patent document 1, patent document 2).

As an example of the "altered form of the WT1 protein-derived partial peptide" in the present invention, an altered peptide having the amino acid sequence of the aforementioned partial peptide, wherein 1 or several (preferably about 1 - 3) amino acids are deleted, substituted or added, which binds to HLA to induce CTL can be mentioned. In the alteration, the amino acid to be inserted or substituted may be a non-natural amino acid other than the 20 kinds of amino acids encoded by the gene. Examples thereof include the following altered forms. Cys-Tyr-Thr-Trp-Asn-Gln-Met-Asn-Leu (CYTWNQMNL) (SEQ ID NO: 4) which is an altered form of WT1₂₃₅₋₂₄₃ peptide (CMTWNQMNL (SEQ ID NO: 6)) (see patent document 4); Arg-Tyr-Phe-Pro-Asn-Ala-Pro-Tyr-Leu (RYFPNAPYL) (SEQ ID NO: 8) which is an altered form of WT1₁₂₆₋₁₃₄ peptide (RMFPNAPYL (SEQ ID NO: 2)) (see patent document 5); Ala-Tyr-Leu-Pro-Ala-Val-Pro-Ser-Leu (AYLPAVPSL) (SEQ ID NO: 9) which is an altered form of WT1₁₀₋₁₈ peptide (ALLPAVPSL (SEQ ID NO: 5)) (see patent document 5); Asn-Tyr-Met-Asn-Leu-Gly-Ala-Thr-Leu (NYMNLGATL) (SEQ ID NO: 11) which is an altered form of WT1₂₃₉₋₂₄₇ peptide (NQMNLGATL (SEQ ID NO: 10)) (see patent document 5); Arg-Tyr-Pro-Ser-Cys-Gln-Lys-Lys-Phe (RYPSCQKKF) (SEQ ID NO: 12) which is an altered form of WT1₄₁₇₋₄₂₅ peptide (RWPSCQKKF (SEQ ID NO: 7)) (see patent document 5); Xaa-Tyr-Thr-Trp-Asn-Gln-Met-Asn-Leu (SEQ ID NO: 13) wherein Xaa is Ser, Ala, Abu, Arg, Lys, Orn, Cit, Leu, Phe or Asn, which is an altered form of WT1₂₃₅₋₂₄₃ peptide (CMTWNQMNL (SEQ ID NO: 6)) (see patent document 6); Xaa-Met-Thr-Trp-Asn-Gln-Met-Asn-Leu (SEQ ID NO: 14) wherein Xaa is Ser or Ala, which is an altered form of WT1₂₃₅₋₂₄₃ peptide (CMTWNQMNL (SEQ ID NO: 6)) (see patent document 6); Phe-Met-Phe-Pro-Asn-Ala-Pro-Tyr-Leu (FMFPNAPYL) (SEQ ID NO: 15), Arg-Leu-Phe-Pro-Asn-Ala-Pro-Tyr-Leu (RLFPNAPYL) (SEQ ID NO: 16), Arg-Met-Met-Pro-Asn-Ala-Pro-Tyr-Leu (RMMPNAPYL) (SEQ ID NO: 17), Arg-Met-Phe-Pro-Asn-Ala-Pro-Tyr-Val (RMFPNAPYV) (SEQ ID NO: 18) or Tyr-Met-Phe-Pro-Asn-Ala-Pro-Tyr-Leu (YMFPNAPYL) (SEQ ID NO: 19), which is an altered form of WT1₁₂₆₋₁₃₄ peptide (RMFPNAPYL (SEQ ID NO: 2)) (see patent document 7); Phe-Leu-Gly-Glu-Gln-Gln-Tyr-Ser-Val (FLGEQQYSV) (SEQ ID NO: 20), Ser-Met-Gly-Glu-Gln-Gln-Tyr-Ser-Val (SMGEQQYSV) (SEQ ID NO: 21) or Ser-Leu-Met-Glu-Gln-Gln-Tyr-Ser-Val (SLMEQQYSV) (SEQ ID NO: 22), which is an altered form of WT1₁₆₇₋₁₉₅ peptide (SLGEQQYSV (SEQ ID NO: 3)) (see patent document 7).

In addition, the "altered form of the WT1 protein-derived partial peptide" in the present invention also includes not only an altered peptide having the amino acid sequence of the aforementioned partial peptide, wherein amino acids are deleted, substituted or added, but also an altered peptide wherein the N terminal of the partial peptide is a cysteine residue and a thiol group of the cysteine residue is chemically modified. Examples thereof include the compounds represented by the following formulas (8) - (13): 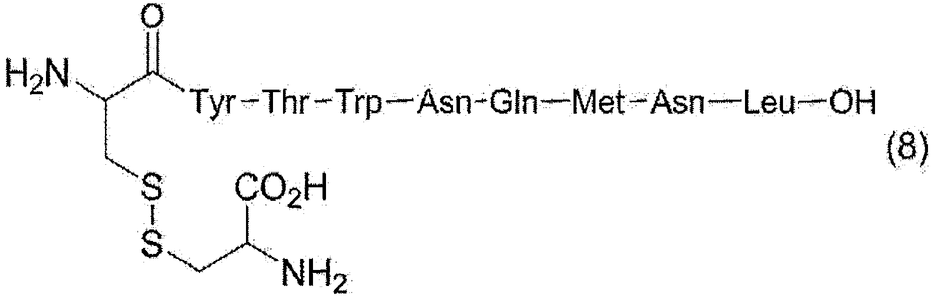 ; 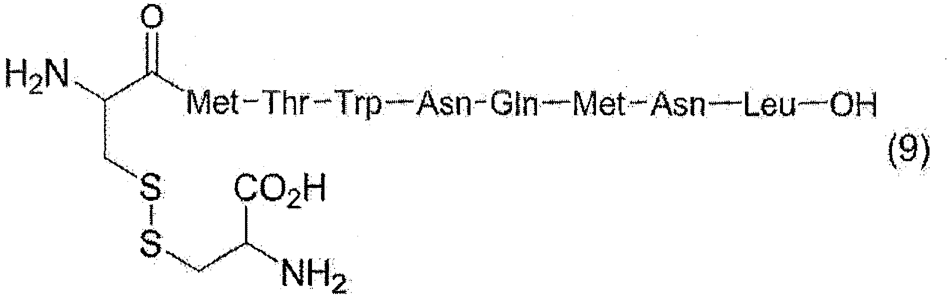 ; 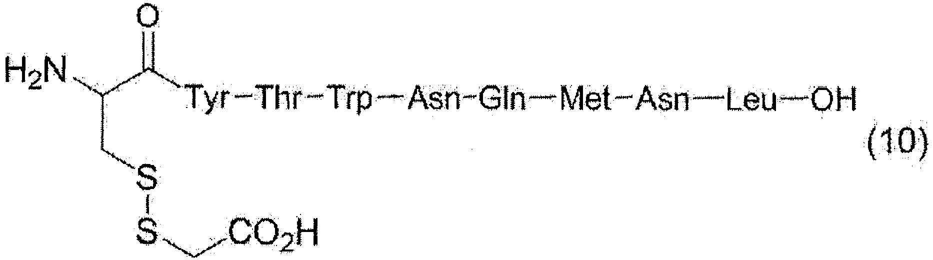 ; 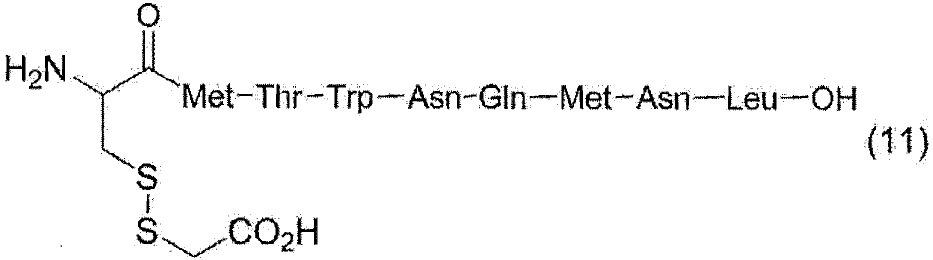 ; 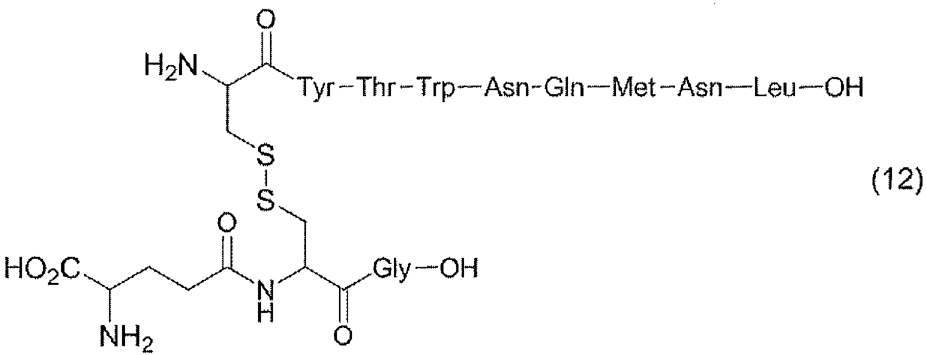 ; and 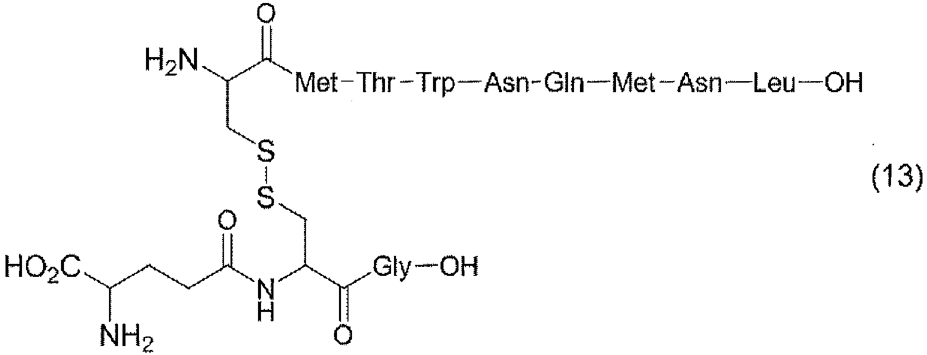 as the altered form of the present invention (see patent document 8).

Furthermore, the "altered form of the WT1 protein-derived partial peptide" in the present invention also includes a dimmer wherein the N terminal of the partial peptide is a cysteine residue, the partial peptide is a monomer and two monomers are dimerized at a thiol group of the cysteine residue via a disulfide bond. As the altered form of the present invention, a compound represented by the following formula (14) : 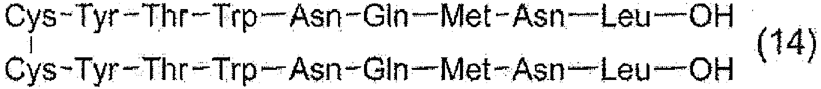 can also be mentioned (see patent document 9).

These "altered forms of the WT1 protein-derived partial peptide" are clearly altered peptides that bind to HLA to induce CTL, and can be produced by a known method (see patent documents 4 - 9).

Preferable examples of the "WT1 protein-derived cancer antigen peptide", namely, "WT1 protein-derived partial peptide or an altered form thereof" of the present invention include a partial peptide consisting of continuous 8 - 12 amino acids in the amino acid sequence of human WT1 protein (SEQ ID NO: 1) and a peptide containing the altered form. A peptide containing any amino acid sequence selected from the following amino acid sequences:
Arg-Met-Phe-Pro-Asn-Ala-Pro-Tyr-Leu (SEQ ID NO: 2),
Ser-Leu-Gly-Glu-Gln-Gln-Tyr-Ser-Val (SEQ ID NO: 3),
Cys-Tyr-Thr-Trp-Asn-Gln-Met-Asn-Leu (SEQ ID NO: 4), and
Ala-Leu-Leu-Pro-Ala-Val-Pro-Ser-Leu (SEQ ID NO: 5)
can be mentioned. Furthermore, a peptide shown by any amino acid sequence selected from the following amino acid sequences:
Arg-Met-Phe-Pro-Asn-Ala-Pro-Tyr-Leu (SEQ ID NO: 2),
Ser-Leu-Gly-Glu-Gln-Gln-Tyr-Ser-Val (SEQ ID NO: 3),
Cys-Tyr-Thr-Trp-Asn-Gln-Met-Asn-Leu (SEQ ID NO: 4), and
Ala-Leu-Leu-Pro-Ala-Val-Pro-Ser-Leu (SEQ ID NO: 5)
can be mentioned, and most preferably, Arg-Met-Phe-Pro-Asn-Ala-Pro-Tyr-Leu (SEQ ID NO: 2) can be mentioned.

The "ether-type additive" in the present invention is ether represented by the formula (1):

R¹-O-R² (1)

[wherein R¹ is a hydrocarbon group having 8 - 24 carbon atoms, and
R² is a group represented by the formula (2): 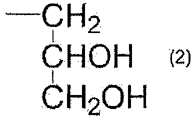
or a group represented by the formula (3):

-(CH₂CH₂O)ₘH (3)

wherein m is an integer of 1 - 18], which is liquid at 20°C.

Of these, as a preferable ether-type additive, a glyceryl ether-type additive represented by the formula (4): 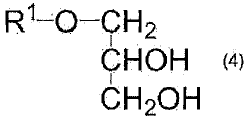 [wherein R¹ is a hydrocarbon group having 8 - 24 carbon atoms], which is liquid at 20°C,
or a polyoxyethylene(POE)ether-type additive represented by the formula (5):

R¹-O-(CH₂CH₂O)ₙH (5)

[wherein R¹ is a hydrocarbon group having 8 - 24 carbon atoms, and
n is an integer of 2 - 18], which is liquid at 20°C, can be mentioned.

As more preferable ether-type additive, a glyceryl ether-type additive represented by the formula (6): 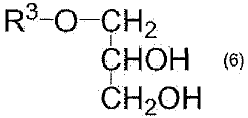 [wherein R³ is a hydrocarbon group having 16 - 18 carbon atoms], which is liquid at 20°C,
or a polyoxyethylene(POE)ether-type additive represented by the formula (7):

R¹-O-(CH₂CH₂O)ₚH (7)

[wherein R¹ is a hydrocarbon group having 8 - 24 carbon atoms, and
p is an integer of 2 - 12], which is liquid at 20°C, can be mentioned.

As used herein, being "liquid at 20°C" means that it can be appropriately warmed to the level not impairing the quality in formulation and used as a liquid, which includes a liquid within the range of 20°C±5°C, and a liquid showing partial precipitation, partial solidification and the like. For example, in an operating manual of the below-mentioned specific additives (commercially available products), those within the range of 20°C±5°C and indicated to be "Liquid", "liquid", "liquid (partially precipitated)", "liquid state", "oil state" or "soft paste state" are included in the "liquid at 20°C" in the present specification.

Among the above, a polyoxyethylene(POE)ether-type additive wherein R¹ in the formula (7) is a branched alkyl group having 8 - 24 carbon atoms, a linear alkenyl group having 8 - 24 carbon atoms or a mixed alkyl group having 8 - 24 carbon atoms is more preferable.

More preferable examples of the ether-type additive include α-monoisostearyl glyceryl ether, monooleyl glyceryl ether, polyoxyethylene isostearyl ether which is liquid at 20°C, polyoxyethylene oleyl ether which is liquid at 20°C, polyoxyethylene alkyl (12 - 14) ether which is liquid at 20°C, polyoxyethylene 2-ethylhexyl ether which is liquid at 20°C and a mixture of these.

Further preferable examples of the ether-type additive include α-monoisostearyl glyceryl ether, monooleyl glyceryl ether, polyoxyethylene isostearyl ether which is liquid at 20°C, polyoxyethylene oleyl ether which is liquid at 20°C, polyoxyethylene alkyl (12 - 14) ether which is liquid at 20°C and a mixture of these.

The most preferable examples of the ether-type additive include α-monoisostearyl glyceryl ether and/or monooleyl glyceryl ether.

The "hydrocarbon group having 8 - 24 carbon atoms" in the present invention is a linear or branched alkyl group having 8 - 24 carbon atoms, a mixed alkyl group having 8 - 24 carbon atoms or a linear or branched alkenyl group having 8 - 24 carbon atoms.

Specific examples of the linear or branched alkyl group having 8 - 24 carbon atoms include an octyl group having 8 carbon atoms, which includes all isomers such as an n-octyl group, an isooctyl group, a sec-octyl group, a tert-octyl group, a neooctyl group, a 2-ethylhexyl group and the like, a nonyl group having 9 carbon atoms, which includes all isomers such as an n-nonyl group, an isononyl group, a sec-nonyl group, a tert-nonyl group, a neononyl group and the like, a decyl group having 10 carbon atoms, which includes all isomers such as an n-decyl group, an isodecyl group, a sec-decyl group, a tert-decyl group, a neodecyl group and the like, an undecyl group having 11 carbon atoms, which includes all isomers such as an n-undecyl group, an isoundecyl group, a sec-undecyl group, a tert-undecyl group, a neoundecyl group and the like, a dodecyl group (lauryl group) having 12 carbon atoms, which includes all isomers such as an n-dodecyl group (n-lauryl group), an isododecyl group (isolauryl group), a sec-dodecyl group (sec-lauryl group), a tert-dodecyl group (tert-lauryl group), a neododecyl group (neolauryl group) and the like, a tridecyl group having 13 carbon atoms, which includes all isomers such as an n-tridecyl group, an isotridecyl group, a sec-tridecyl group, a tert-tridecyl group, a neotridecyl group and the like, a tetradecyl group (myristyl group) having 14 carbon atoms, which includes all isomers such as an n-tetradecyl group (n-myristyl group), an isotetradecyl group (isomyristyl group), a sec-tetradecyl group (sec-myristyl group), a tert-tetradecyl group (tert-myristyl group), a neotetradecyl group (neomyristyl group) and the like, a pentadecyl having 15 carbon atoms, which includes all isomers such as an n-pentadecyl group, an isopentadecyl group, a sec-pentadecyl group, a tert-pentadecyl group, a neopentadecyl group and the like, a hexadecyl group (cetyl group) having 16 carbon atoms, which includes all isomers such as an n-hexadecyl group (n-cetyl group), an isohexadecyl group (isocetyl group), a sec-hexadecyl group (sec-cetyl group), a tert-hexadecyl group (tert-cetyl group), a neohexadecyl group (neocetyl group) and the like, a heptadecyl group having 17 carbon atoms, which includes all isomers such as an n-heptadecyl group, an isoheptadecyl group, a sec-heptadecyl group, a tert-heptadecyl group, a neoheptadecyl group and the like, an octadecyl group (stearyl group) having 18 carbon atoms, which includes all isomers such as an n-octadecyl group (n-stearyl group), an isooctadecyl group (isostearyl group), a sec-octadecyl group (sec-stearyl group), a tert-octadecyl group (tert-stearyl group), a neooctadecyl group (neostearyl group) and the like, a nonadecyl group having 19 carbon atoms, which includes all isomers such as an n-nonadecyl group, an isononadecyl group, a sec-nonadecyl group, a tert-nonadecyl group, a neononadecyl group and the like, an icosyl group having 20 carbon atoms, which includes all isomers such as an n-icosyl group, an isoicosyl group, a sec-icosyl group, a tert-icosyl group, a neoicosyl group and the like, a henicosyl group having 21 carbon atoms, which includes all isomers such as an n-henicosyl group, an isohenicosyl group, a sec-henicosyl group, a tert-henicosyl group, a neohenicosyl group and the like, a docosyl group having 22 carbon atoms, which includes all isomers such as an n-docosyl group, an isodocosyl group, a sec-docosyl group, a tert-docosyl group, a neodocosyl group and the like, a tricosyl group having 23 carbon atoms, which includes all isomers such as an n-tricosyl group, an isotricosyl group, a sec-tricosyl group, a tert-tricosyl group, a neotricosyl group and the like, and a tetracosyl group having 24 carbon atoms, which includes all isomers such as an n-tetracosyl group, an isotetracosyl group, a sec-tetraosyl group, a tert-tetraosyl group, a neotetracosyl group, a neodecyltetradecyl group and the like and the like. Of these, an alkyl group having 12 - 18 carbon atoms is preferable, and an alkyl group having 16 - 18 carbon atoms is more preferable.

The ether-type additive in the present invention is also defined by property requirement that it is liquid at 20°C, in addition to the structure requirements of the formula (1). Therefore, for example, when R² in the formula (1) is a group represented by the formula (2), namely, a glyceryl ether-type additive, the formula (1) wherein R¹ is a linear alkyl group having 18 carbon atoms, i.e., a stearyl group, namely, stearyl glyceryl ether which is solid at 20°C is not included in the ether-type additive of the present invention. Similarly, glyceryl ether-type additive wherein R¹ is a cetyl group of a linear alkyl group having 16 carbon atoms, namely, cetyl glyceryl ether, is also solid at 20°C, and therefore, it is not included in the ether-type additive of the present invention. Whether an ether-type additive is liquid at 20°C can be easily determined from the information such as catalog and the like supplied by the maker selling the ether-type additives, as mentioned below.

Specific examples of the linear or branched alkenyl group having 8 - 24 carbon atoms include an octenyl group having 8 carbon atoms, which includes all isomers such as an n-octenyl group, an isooctenyl group, a sec-octenyl group, a tert-octenyl group, a neooctenyl group and the like, a nonenyl group having 9 carbon atoms, which includes all isomers such as an n-nonenyl group, an isononenyl group, a sec-nonenyl group, a tert-nonenyl group, a neononenyl group and the like, a decenyl group having 10 carbon atoms, which includes all isomers such as an n-decenyl group, an isodecenyl group, a sec-decenyl group, a tert-decenyl group, a neodecenyl group and the like, an undecenyl group having 11 carbon atoms, which includes all isomers such as an n-undecenyl group, an isoundecenyl group, a sec-undecenyl group, a tert-undecenyl group, a neoundecenyl group and the like, a dodecenyl group having 12 carbon atoms, which includes all isomers such as an n-dodecenyl group, an isododecenyl group, a sec-dodecenyl group, a tert-dodecenyl group, a neododecenyl group and the like, a tridecenyl group having 13 carbon atoms, which includes all isomers such as an n-tridecenyl group, an isotridecenyl group, a sec-tridecenyl group, a tert-tridecenyl group, a neotridecenyl group and the like, a tetradecenyl group having 14 carbon atoms, which includes all isomers such as an n-tetradecenyl group, an isotetradecenyl group, a sec-tetradecenyl group, a tert-tetradecenyl group, a neotetradecenyl group and the like, a pentadecenyl group having 15 carbon atoms, which includes all isomers such as an n-pentadecenyl group, an isopentadecenyl group, a sec-pentadecenyl group, a tert-pentadecenyl group, a neopentadecenyl group and the like, a hexadecenyl having 16 carbon atoms, which includes all isomers such as an n-hexadecenyl group, an isohexadecenyl group, a sec-hexadecenyl group, a tert-hexadecenyl group, a neohexadecenyl group and the like, a heptadecenyl group having 17 carbon atoms, which includes all isomers such as an n-heptadecenyl group, an isoheptadecenyl group, a sec-heptadecenyl group, a tert-heptadecenyl group, a neoheptadecenyl group and the like, an octadecenyl group having 18 carbon atoms, which includes all isomers such as an n-octadecenyl group, an isooctadecenyl group, a sec-octadecenyl group, a tert-octadecenyl group, a neooctadecenyl group, a 9-octadecenyl group, a linoleyl group (cis-9,cis-12-octadecadienyl group), an oleyl group and the like, a nonadecenyl group having 19 carbon atoms, which includes all isomers such as an n-nonadecenyl group, an isononadecenyl group, a sec-nonadecenyl group, a tert-nonadecenyl group, a neononadecenyl group and the like, an icosenyl group having 20 carbon atoms, which includes all isomers such as an n-icosenyl group, an isoicosenyl group, a sec-icosenyl group, a tert-icosenyl group, a neoicosenyl group and the like, a henicosenyl group having 21 carbon atoms, which includes all isomers such as an n-henicosenyl group, an isohenicosenyl group, a sec-henicosenyl group, a tert-henicosenyl group, a neohenicosenyl group and the like, a docosenyl group having 22 carbon atoms, which includes all isomers such as an n-docosenyl group, an isodocosenyl group, a sec-docosenyl group, a tert-docosenyl group, a neodocosenyl group and the like, a tricosenyl group having 23 carbon atoms, which includes all isomers such as an n-tricosenyl group, an isotricosenyl group, a sec-tricosenyl group, a tert-tricosenyl group, a neotricosenyl group and the like, and a tetracosenyl group having 24 carbon atoms, which includes all isomers such as an n-tetracosenyl group, an isotetracosenyl group, a sec-tetracosenyl group, a tert-tetracosenyl group, a neotetracosenyl group and the like and the like. Of these, an alkenyl group having 12 - 18 carbon atoms is preferable, and an alkenyl group having 16 - 18 carbon atoms is more preferable.

The "hydrocarbon group having 16 - 18 carbon atoms" in the present invention is, among the aforementioned "hydrocarbon group having 8 - 24 carbon atoms", a linear or branched alkyl group having 16 - 18 carbon atoms, a mixed alkyl group having 16 - 18 carbon atoms or a linear or branched alkenyl group having 16 - 18 carbon atoms.

The "mixed" in the present invention means that plural hydrocarbon groups are contained rather than a single hydrocarbon group. For example, alkyl (12 - 14) means that plural alkyl groups of a dodecyl group having 12 carbon atoms, a tridecyl group having 13 carbon atoms, and a tetradecyl group having 14 carbon atoms are contained.

When R² in the formula (1) is a polyoxyethylene group represented by the formula (3), namely, a POE ether-type additive, "m" in the formula (3) is an integer showing an average mole number of added ethylene oxide in the POE ether-type additive obtained by addition polymerization of ethylene oxide. m is an integer of 1 - 18, preferably an integer of 2 - 18, more preferably an integer of 2 - 12, further preferably an integer of 2 - 10, and most preferably an integer of 2 - 5.

"n" in the POE ether-type additive represented by the formula (5) and "p" in the POE ether-type additive represented by the formula (7) are also each independently an integer showing an average mole number of added ethylene oxide in the POE ether-type additive obtained by addition polymerization of ethylene oxide. n is an integer of 2 - 18, preferably an integer of 2 - 12, more preferably an integer of 2 - 10, and most preferably an integer of 2 - 5. p is an integer of 2 - 12, preferably an integer of 2 - 10, and most preferably an integer of 2 - 5.

Of the ether-type additives represented by the formula (1), R¹ is a hydrocarbon group having 8 - 24 carbon atoms. When R² of the additive is a glyceryl group represented by the formula (2), namely, a glyceryl ether-type additive, R¹ is most preferably an isostearyl group or an oleyl group. When R² is a polyoxyethylene group represented by the formula (3), namely, a POE ether-type additive, R¹ is preferably an n-decyl group, an n-dodecyl group (n-lauryl group), an n-tridecyl group, a 2-ethylhexyl group, an isodecyl group, an isocetyl group, an isostearyl group, a decyltetradecyl group, an oleyl group, a dodecyl group, a tridecyl group, a tetradecyl group, a pentadecyl group or a hexadecyl group. Among these, an isostearyl group, an oleyl group, a dodecyl group, a tridecyl group or a tetradecyl group is most preferable. That is, as the ether-type additive in the present invention, preferred is α-monoisostearyl glyceryl ether, monooleyl glyceryl ether, polyoxyethylene isostearyl ether which is liquid at 20°C, polyoxyethylene oleyl ether which is liquid at 20°C, or polyoxyethylene alkyl (12 - 14) ether which is liquid at 20°C. The transdermal preparation of the present invention may contain one or more kinds of these preferable ether-type additives.

As one embodiment of the ether-type additive in the present invention, α-monoisostearyl glyceryl ether, monooleyl glyceryl ether, polyoxyethylene isostearyl ether which is liquid at 20°C, polyoxyethylene oleyl ether which is liquid at 20°C, polyoxyethylene alkyl (12 - 14) ether which is liquid at 20°C, polyoxyethylene 2-ethylhexyl ether which is liquid at 20°C, and a mixture of two or more kinds of these can be mentioned.

The α-monoisostearyl glyceryl ether to be used in the present invention is listed in the Japanese Pharmaceutical Excipients, and is commercially available as PENETOL GE-IS (manufactured by Kao Corporation). α-Monoisostearyl glyceryl ether is liquid at 20°C. Although it is sometimes solidified partially at a low temperature, it is preferably heated as appropriate and used as a liquid for formulation.

Monooleyl glyceryl ether to be used in the present invention is listed in the Japanese Standard of Quasi-drug Ingredients, and is commercially available as selachyl alcohol (manufactured by Nikko Chemicals Co., Ltd.). Monooleyl glyceryl ether is liquid at 20°C.

While polyoxyethylene isostearyl ether is listed in the Japanese Standard of Quasi-drug Ingredients and is commercially available, the polyoxyethylene isostearyl ether to be used in the present invention may be any as long as it is liquid at 20°C (unless otherwise indicated, the state of each polyoxyethylene isostearyl ether described below is that at 20±5°C.). Polyoxyethylene isostearyl ether is generally obtained by addition polymerization of ethylene oxide mainly to isostearyl alcohol, and the average mole number of added ethylene oxide is 5, 10, 15, 20 or 25. Of these, polyoxyethylene isostearyl ether having an average mole number of added ethylene oxide of 5 is a liquid. Polyoxyethylene isostearyl ether having an average mole number of added ethylene oxide of 10 or 15 is a soft wax state, which is preferably heated as appropriate and used as a liquid for formulation. Preferable examples of polyoxyethylene isostearyl ether to be used in the present invention include polyoxyethylene isostearyl ether having an average mole number of added ethylene oxide of not more than 15 (more preferably not more than 5). Among the commercially available polyoxyethylene isostearyl ethers, EMALEX 1805 (manufactured by Nihon Emulsion Co., Ltd., average mole number of added ethylene oxide=5, soft paste state) and FINESURF FO-40-90 (manufactured by AOKI OIL INDUSTRIAL Co., Ltd., average mole number of added ethylene oxide=4, liquid) are preferable.

Polyoxyethylene oleyl ether is listed in the Japanese Standard of Quasi-drug Ingredients and Japanese Pharmaceutical Excipients, and is commercially available. The polyoxyethylene oleyl ether to be used in the present invention may be any as long as it is liquid at 20°C (unless otherwise indicated, the state of each polyoxyethylene oleyl ether described below is that at 20±5°C.). Polyoxyethylene oleyl ether is generally obtained by addition polymerization of ethylene oxide to higher aliphatic alcohol mainly composed of oleyl alcohol, and the average mole number of added ethylene oxide is 2, 3, 4, 5, 6, 7, 8, 9, 10, 14, 15, 20, 23 or 50. Of these, polyoxyethylene oleyl ether having an average mole number of added ethylene oxide of any of 2 to 10 is a liquid. Manufactures indicate that an average mole number of added ethylene oxide of 10 means a liquid containing solid, and an average mole number of added ethylene oxide of 15 means a paste state. These polyoxyethylene oleyl ethers are preferably warmed as appropriate and used as a liquid for formulation. As preferable polyoxyethylene oleyl ether to be used in the present invention, polyoxyethylene oleyl ether having an average mole number of added ethylene oxide of not more than 10 can be mentioned and, more preferably, polyoxyethylene oleyl ether having an average mole number of added ethylene oxide of 2 to 7 can be mentioned. Among the commercially available polyoxyethylene oleyl ethers, BLAUNON EN-1502, BLAUNON EN-1504 and BLAUNON EN-905 (all manufactured by AOKI OIL INDUSTRIAL Co., Ltd., average mole number of added ethylene oxide (in the above order)=2, 4, 5, all are liquids), EMALEX 503 and EMALEX 505H (both manufactured by Nihon Emulsion Co., Ltd., average mole number of added ethylene oxide (in the above order)=3, 5, both are oil), Newcol 1203 and Newcol 1204 (both manufactured by NIPPON NYUKAZAI Co., Ltd., average mole number of added ethylene oxide (in the above order)=3, 4, both are liquid), EMULMIN CO-50 and EMULMIN CO-100 (both manufactured by Sanyo Chemical Industries, Ltd., average mole number of added ethylene oxide (in the above order)=5, 10, both are liquid), and NIKKOL BO-2V and NIKKOL BO-7V (both manufactured by Nikko Chemicals Co., Ltd., average mole number of added ethylene oxide (in the above order)=2, 7, both are liquid) are preferable.

Polyoxyethylene alkyl (12 - 14) ether is listed in the Japanese Standard of Quasi-drug Ingredients and Japanese Pharmaceutical Excipients, and is commercially available. Polyoxyethylene alkyl (12 - 14) ether to be used in the present invention may be any as long as it is liquid at 20°C (unless otherwise indicated, the state of each polyoxyethylene alkyl (12 - 14) ether described below is that at 20±5°C.). Polyoxyethylene alkyl (12 - 14) ether is generally obtained by addition polymerization of ethylene oxide mainly to alcohol containing an alkyl group having 12 - 14 carbon atoms, has an average mole number of added ethylene oxide of 3, 5, 7, 9 or 12 and is a liquid. As commercially available polyoxyethylene alkyl (12 - 14) ethers, NOIGEN ET-65, NOIGEN ET-95, NOIGEN ET-115, NOIGEN ET-135 and NOIGEN ET-165 (all manufactured by DKS Co., Ltd., average mole number of added ethylene oxide (in the above order)=3, 5, 7, 9, 12, all are liquid), SANNONIC SS-70, SANNONIC SS-90 and SANNONIC SS-120 (all manufactured by Sanyo Chemical Industries, Ltd., average mole number of added ethylene oxide (in the above order)=7, 9, 12, all are liquid), and NIKKOL BT-3, NIKKOL BT-5, NIKKOL BT-7, NIKKOL BT-9 and NIKKOL BT-12 (all manufactured by Nikko Chemicals Co., Ltd., average mole number of added ethylene oxide (in the above order)=3, 5, 7, 9, 12, all are liquid) are preferable.

In the ether-type additive represented by the formula (1) in the present invention, when R² is a group represented by the formula (2), namely, a glyceryl ether-type additive, examples thereof include α-monoisostearyl glyceryl ether, monooleyl glyceryl ether, monoisocetyl glyceryl ether, n-cetyl glyceryl ether and n-hexadecenyl glyceryl ether to be the main components of alkyl glyceryl ether in chimaera liver oil, and isodecyl glyceryl ether entered in the component list of Cosmetic Industry Association, which are liquid at 20°C. As the glyceryl ether-type additive of the present invention, α-monoisostearyl glyceryl ether, monooleyl glyceryl ether, and a mixture thereof are preferable.

Among the ether-type additives represented by the formula (1) in the present invention, when R² is a group represented by the formula (3), namely, POE ether-type additive, examples thereof include the aforementioned polyoxyethylene isostearyl ether which is liquid at 20°C, polyoxyethylene oleyl ether which is liquid at 20°C, and polyoxyethylene alkyl (12 - 14) ether which is liquid at 20°C, as well as the following a) - 1) ;
a) polyoxyethylene decyl ether: Commercially available as FINESURF D-1303, FINESURF D-1305, FINESURF D-1307 and FINESURF D-1310 (all manufactured by AOKI OIL INDUSTRIAL Co., Ltd.). Of these, one having an average mole number of added ethylene oxide of 3, 5 or 10 is liquid at 20°C and preferable;
b) polyoxyethylene lauryl ether: Commercially available as BLAUNON EL-1502.2, BLAUNON EL-1503P, BLAUNON EL-1505, BLAUNON EL-1505P, BLAUNON EL-1507, BLAUNON EL-1508P, BLAUNON EL-1509P and BLAUNON EL-1509.5 (all manufactured by AOKI OIL INDUSTRIAL Co., Ltd.), DKS NL-15, DKS NL-30, DKS NL-40, DKS NL-50, DKS NL-60 and DKS NL-70 (all manufactured by DKS Co. Ltd.), EMALEX 703, EMALEX 705, EMALEX 707 and EMALEX 709 (all manufactured by Nihon Emulsion Co., Ltd.), EMULMIN NL-70, EMULMIN LS-80, EMULMIN LS-90, EMULMIN NL-100 and EMULMIN NL-110 (all manufactured by Sanyo Chemical Industries, Ltd.), NIKKOL BL-2, NIKKOL BL-4.2 and NIKKOL BL-9EX (all manufactured by Nikko Chemicals Co., Ltd.), and Emulgen 104P and Emulgen 106 (all manufactured by Kao Corporation). Of these, one having an average mole number of added ethylene oxide of not more than 9 is liquid at 20°C and preferable;
c) polyoxyethylene tridecyl ether: Commercially available as FINESURF TD-30, FINESURF TD-50, FINESURF TD-65, FINESURF TD-70, FINESURF TD-75, FINESURF TD-80, FINESURF TD-85, FINESURF TD-90 and FINESURF TD-100 (all manufactured by AOKI OIL INDUSTRIAL Co., Ltd.), NOIGEN TDS-30, NOIGEN TDS-50, NOIGEN TDS-70, NOIGEN TDS-80, NOIGEN TDS-100 and NOIGEN TDS-120 (all manufactured by DKS Co. Ltd.), and Newcol 1305 (manufactured by NIPPON NYUKAZAI Co., Ltd.). Of these, one having an average mole number of added ethylene oxide of not more than 10 is liquid at 20°C and preferable;
d) polyoxyethylene 2-ethylhexyl ether: Commercially available as BLAUNON EH-2, BLAUNON EH-4, BLAUNON EH-6 and BLAUNON EH-11 (all manufactured by AOKI OIL INDUSTRIAL Co., Ltd.), and Newcol 1004, Newcol 1006 and Newcol 1008 (all manufactured by NIPPON NYUKAZAI Co., Ltd.). Of these, one having an average mole number of added ethylene oxide of not more than 8 is liquid at 20°C and preferable;
e) polyoxyethylene isodecyl ether: Commercially available as FINESURF D-35, FINESURF D-60, FINESURF D-65, FINESURF D-85, SAFETYCUT ID-1033, SAFETYCUT ID-1055 and SAFETYCUT ID-1061 (all manufactured by AOKI OIL INDUSTRIAL Co., Ltd.), and NOIGEN SD-30, NOIGEN SD-60, NOIGEN SD-70, NOIGEN SD-80 and NOIGEN SD-110 (all manufactured by DKS Co. Ltd.). Of these, one having an average mole number of added ethylene oxide of not more than 8.5 is liquid at 20°C and preferable;
f) polyoxyethylene isocetyl ether: Commercially available as EMALEX 1605 and EMALEX 1610 (all manufactured by Nihon Emulsion Co., Ltd.). Of these, one having an average mole number of added ethylene oxide of not more than 10 is soft paste state at 20°C and preferable;
g) polyoxyethylene decyltetradecyl ether: Commercially available as EMALEX 2405 and EMALEX 2410 (all manufactured by Nihon Emulsion Co., Ltd.). Of these, one having an average mole number of added ethylene oxide of not more than 5 is soft paste state at 20°C and preferable;
h) polyoxyethylene synthetic alcohol (C12 - 13) ether: Commercially available as FINESURF NE-20, FINESURF NE-50 and FINESURF NE-100 (all manufactured by AOKI OIL INDUSTRIAL Co., Ltd.), and Newcol 2302 and Newcol 2303 (all manufactured by NIPPON NYUKAZAI Co., Ltd.). Of these, one having an average mole number of added ethylene oxide of not more than 10 is liquid at 20°C and preferable;
i) polyoxyethylene synthetic alcohol (C14 - 15) ether: Commercially available as BLAUNON OX-33 and BLAUNON OX-70 (both manufactured by AOKI OIL INDUSTRIAL Co., Ltd.). Of these, one having an average mole number of added ethylene oxide of not more than 7 is liquid at 20°C and preferable;
j) polyoxyethylene alkyl (C12 - 15) ether: Commercially available as NIKKOL BD-4 (manufactured by Nikko Chemicals Co., Ltd.). The average mole number of added ethylene oxide of 4 is liquid at 20°C;
k) polyoxyethylene secondary alcohol ether: Commercially available as FINESURF 230, FINESURF 250, FINESURF 270 and FINESURF 290 (all manufactured by AOKI OIL INDUSTRIAL Co., Ltd.), and Newcol NT-3, Newcol NT-5, Newcol NT-7 and Newcol NT-9 (all manufactured by NIPPON NYUKAZAI Co., Ltd.). Of these, one having an average mole number of added ethylene oxide of not more than 9 is liquid at 20°C and preferable;
l) polyoxyethylene oleylcetyl ether: Commercially available as NOIGEN ET-69 and NOIGEN ET-109 (both manufactured by DKS Co., Ltd.), and EMULMIN 40 and EMULMIN 50 (both manufactured by Sanyo Chemical Industries, Ltd.). Of these, one having an average mole number of added ethylene oxide of not more than 5 is liquid at 20°C and preferable.

An ether-type additive represented by the formula (1) in the present invention is commercially available as mentioned above, or can be produced by a known method by using a known compound as a starting material. For example, it can be produced by the methods shown in production methods 1 and 2 below, a method similar to the following production method, or an appropriate combination of the synthesis methods well known to those of ordinary skill in the art.
Production method 1: A production method of the ether-type additive of the formula (4) includes known methods such as a condensation reaction of straight chain or branched alkyl halide having 8 - 24 carbon atoms or alkenyl halide (hydrocarbon halide) and glycerol alcholate and the like. The synthesis method may be a known method, wherein glycerol alcholate is prepared in advance from glycerol and sodium hydroxide or potassium hydroxide and the like, which is then reacted with hydrocarbon halide. The reaction temperature is suitably 100°C - 200°C, and the reaction time is suitably 1 hr to 5 hr. Since the obtained product generally contains an inorganic salt such as sodium chloride and the like, it is purified by washing with water, and further by recrystallization from methanol or ethanol. In other synthesis method, glycidyl ether is prepared from higher alcohol such as isooctanol, octanol, lauryl alcohol, oleyl alcohol, stearyl alcohol, behenyl alcohol, isostearyl alcohol and the like and epichlorohydrin, and an epoxy bond therein is opened to give the additive. Since the obtained resultant product similarly contains an inorganic salt such as sodium chloride and the like, it is purified by washing with water, and further by recrystallization from methanol or ethanol.
Production method 2: A production method of the ether-type additive of the formula (5) includes a method comprising synthesizing higher alcohol added with 1 or 2 mol of ethylene oxide at high purity, and adding ethylene oxide to a desired number of moles according to a general method, and a method comprising adding ethylene oxide to higher alcohol in the presence of an acidic catalyst, a polyvalent metal catalyst (calcium oxide, magnesium oxide), or/and a clay mineral catalyst at 100 - 150°C under low pressure.

The "transdermal preparation" in the present invention is not limited as long as it is a preparation permitting transdermal administration. As the dosage form of the transdermal preparation, a dosage form conventionally used for external preparation, such as patches preparation, ointments, creams, gels, gel creams, lotions, sprays for cutaneous application, aerosols, liniments, microneedle preparation and the like, can be used. The preparation can be used as an external preparation in any dosage form. Among these, a patches preparation is preferable. The patches preparation refers to preparations in general that can be adhered to the skin, and includes, for example, tapes preparation, patches, cataplasms preparation, plaster preparation and the like. Among these, tapes preparation and patches are particularly preferable.

The transdermal preparation of the present invention can be produced by a general method by adding a suitable amount of at least one kind of ether-type additive selected from the group consisting of α-monoisostearyl glyceryl ether, monooleyl glyceryl ether, polyoxyethylene isostearyl ether which is liquid at 20°C, polyoxyethylene oleyl ether which is liquid at 20°C, and polyoxyethylene alkyl (12 - 14) ether which is liquid at 20°C to the preparation. When the solubility of these particular ether-type additives and a base is not good, a solvent can also be used as appropriate to improve the solubility. As the transdermal preparation of the present invention, a patches preparation is explained in more detail now.

A patches preparation can contain the above-mentioned particular ether-type additive in the adhesive layer of the preparation together with a drug (WT1 protein-derived cancer antigen peptide) and an adhesive. Furthermore, where necessary, the below-mentioned pharmaceutically acceptable formulation components used for the production of patches preparations may also be added. The amount of the ether-type additive to be contained in the patches preparation of the present invention is generally not less than 0.01 wt%, preferably not less than 0.1 wt%, more preferably not less than 0.3 wt%, further preferably not less than 0.5 wt%, particularly preferably not less than 1 wt% (e.g., not less than 1.5 wt%, not less than 2 wt%, not less than 2.5 wt%), relative to the total amount of the adhesive layer. In addition, the amount to be added is generally not more than 50 wt%, preferably not more than 40 wt%, more preferably not more than 30 wt%, further preferably not more than 25 wt%, particularly preferably not more than 20 wt% (e.g., not more than 18 wt%, not more than 15 wt%, not more than 12 wt%), relative to the total amount of the adhesive layer.

The adhesive layer in the present invention is a layer containing a WT1 protein-derived cancer antigen peptide, which is formed on a support. The adhesive layer contains the peptide, the above-mentioned particular ether-type additive and an adhesive. Where necessary, other additive can also be contained.

The adhesive additive layer in the present invention is a layer not substantially containing a WT1 protein-derived cancer antigen peptide but containing the above-mentioned particular ether-type additive and an adhesive. Where necessary, other additive can also be contained.

The adhesive additive layer can contain any additive (the above-mentioned particular ether-type additive, other additive) that can be contained in the adhesive layer; however, the additive does not need to be the same as the additive contained in the adhesive layer. In other words, the above-mentioned particular ether-type additives contained in the adhesive additive layer and the adhesive layer may or may not be the same. The additive contained in the adhesive additive is preferably the same component as the adhesive layer, and more preferably the same amount of the same component as the adhesive layer.

In the present specification, "not substantially containing WT1 protein-derived cancer antigen peptide" means either a WT1 protein-derived cancer antigen peptide is not contained at all or a WT1 protein-derived cancer antigen peptide is contained at a level not influencing the efficacy. For example, a peptide in about 1/10 of the WT1 protein-derived cancer antigen peptide contained in the adhesive layer may be contained.

When an adhesive additive layer is formed on a support in a patches preparation, it is generally formed in the order of the adhesive additive layer and the adhesive layer, from the support side. The adhesive additive layer and the adhesive layer are preferably continuous layers.

In the present invention, a simple indication of "wt%" means wt% relative to the total weight of the adhesive layer substantially free of a solvent and the like due to drying and the like as 100 wt%.

As an adhesive to be used for the patches preparation of the present invention can be appropriately selected from known ones in consideration of the skin safety, drug releaseability, attachment to skin and the like. Preferable examples of the adhesive include silicone adhesive, rubber adhesive, acrylic adhesive and the like.

Examples of the silicone adhesive include those containing silicone rubber as a main component, such as polydimethyl siloxane, diphenylsiloxane and the like.

Examples of the rubber adhesive include natural rubber, polyisopropylene rubber, polyisobutylene, styrene-butadiene copolymer, styrene-isopropylene copolymer, styrene-isoprene-styrene block copolymer and the like.

Examples of the acrylic adhesive include (co)polymers mainly comprising (meth)acrylic acid alkyl ester. Specific examples include polymers mainly comprising acrylic acid alkyl ester, polymers mainly comprising methacrylic acid alkyl ester, copolymers mainly comprising acrylic acid alkyl ester, copolymers mainly comprising methacrylic acid alkyl ester, and copolymers mainly comprising acrylic acid alkyl ester and methacrylic acid alkyl ester. The (co)polymer may be a copolymer of two or more kinds of (meth)acrylic acid alkyl ester as mentioned above, or may be a copolymer of (meth)acrylic acid alkyl ester and (a) functional monomer(s) capable of copolymerizing with (meth)acrylic acid alkyl ester.

Here, the "(meth)acrylic acid" means "acrylic acid or methacrylic acid", or "acrylic acid and/or methacrylic acid", and the "(co)polymer" means "polymer or copolymer", or "polymer and/or copolymer".

Examples of the (meth)acrylic acid alkyl ester include those obtained by esterification with linear or branched alkyl having 1 - 18 carbon atoms, and specifically include (meth)acrylic acid methyl ester, (meth)acrylic acid butyl ester, (meth)acrylic acid hexyl ester, (meth)acrylic acid octyl ester, (meth)acrylic acid nonyl ester, (meth)acrylic acid decyl ester and the like.

Examples of the functional monomer include a monomer having a hydroxyl group ((meth)acrylic acid hydroxyethyl ester etc.), a monomer having a carboxyl group (butyl maleate, crotonic acid etc.), a monomer having an amido group ((meth)acrylamide etc.), a monomer having an amino group (dimethylamino(meth)acrylate etc.), a monomer having a pyrrolidone ring (N-vinyl-2-pyrrolidone etc.) and the like.

The acrylic adhesive in the present invention may be used singly or in a combination of two or more kinds thereof. In addition, it may be a mixture with other adhesive. Examples of the other adhesive include silicone adhesive, rubber adhesive and the like. Specific preferable examples of the acrylic adhesive include, but are not limited to, acrylic acid-acrylic acid octyl ester copolymer, (meth)acrylate-vinyl acetate copolymer, 2-ethylhexyl acrylate-vinylpyrrolidone copolymer, 2-ethylhexyl acrylate-2-ethylhexyl methacrylate-dodecyl methacrylate copolymer, ethyl acrylate-methyl methacrylate copolymer, acrylic acid-silk fibroin copolymer, methyl acrylate-2-ethylhexyl acrylate copolymer and the like, and include commercially available products such as "POLYTHICK 410-SA" manufactured by Sanyo Chemical Industries, Ltd., "Oribain BPS-4849-40" manufactured by TOYO INK CO., LTD., "DURO-TAK 87-2194", "DURO-TAK 387-2516" and "DURO-TAK 387-2287" manufactured by Henkel, "MAS811", "MAS683" and "MAS955" manufactured by CosMED Pharmaceutical Co. Ltd. and the like.

To afford appropriate adhesiveness to the skin, moreover, a curing agent may also be added where necessary. Examples of the curing agent include commercially available products such as "POLYTHICK SC-75" manufactured by Sanyo Chemical Industries, Ltd., "BHS8515" manufactured by TOYO INK CO., LTD. and the like. The amount thereof to be added can be appropriately selected according to the property of the adhesive, which is, for example, about 0.001 - 0.05 part by weight relative to 1 part by weight of the adhesive.

The amount of the adhesive to be contained in the patches preparation of the present invention is a balance after removing WT1 protein-derived cancer antigen peptide, particular ether-type additives, and various formulation components mentioned below, which are added as necessary, from the adhesive layer, and is the amount necessary for completing the adhesive layer. Thus, for example, when the adhesive layer comprises about 10 wt% of WT1 protein-derived cancer antigen peptide and 20 wt% of ether-type additive, the amount of the adhesive is about 70 wt%.

The adhesiveness of the adhesive to be used here is of the level employed for medical patches preparations, and intended to mean adhesiveness of the level permitting easy adhesion to the skin and causing no particular problem in peeling off.

While the adhesive additive layer does not need to contain the same adhesive as the adhesive contained in the adhesive layer, the adhesive contained in the adhesive additive is preferably the same component as the adhesive layer. The amount of the adhesive to be added to the adhesive additive layer is a balance after removing, from the adhesive additive layer, particular ether-type additive, and various formulation components mentioned below, which are added for formulation as necessary, from the adhesive additive layer, and is the amount necessary for completing the adhesive additive layer.

The amount of the WT1 protein-derived cancer antigen peptide to be added to the patches preparation of the present invention when the WT1 protein-derived cancer antigen peptide is in the form of a salt is generally about 0.1 - 40 wt%, preferably about 0.1 - 30 wt%, more preferably about 0.1 - 20 wt%, further preferably about 0.1 - 15 wt%, most preferably about 0.1 - 10 wt%, and also preferably about 0.5 - 40 wt%, more preferably about 0.5 - 30 wt%, further preferably about 0.5 - 20 wt%, moreover preferably 0.5 - 15 wt%, most preferably about 0.5 - 10 wt%, and also preferably about 1 - 30 wt%, more preferably about 1 - 20 wt%, further preferably about 1 - 15 wt%, most preferably about 1 - 10 wt%, in terms of a free base and the like, of the adhesive layer as 100 wt%, though subject to change depending on the area of the patches preparation. Here, in terms of a free base and the like means that, when WT1 protein-derived cancer antigen peptide is in the form of a salt or contains crystal water, the amount corresponding to the salt or the crystal water is not included in the weight of WT1 protein-derived cancer antigen peptide. In other words, it means that the amount of a salt or a hydrate of WT1 protein-derived cancer antigen peptide thereof is calculated by converting the weight thereof to the weight of an equimolar amount of WT1 protein-derived cancer antigen peptide (free base non-hydrate).

As a conventional pharmaceutically acceptable formulation component contained in the patches preparation of the present invention as necessary, any component can be used as long as its addition does not cause any particular problem and the addition is necessary, and, for example, stabilizer, tackifier, plasticizer, flavor, filler, thickener, and the like can be mentioned.

Examples of the stabilizer include, but are not limited to, ascorbic acid, sodium alginate, propylene glycol alginate, dibutylhydroxytoluene, butylhydroxyanisole, tocopherol acetate, tocopherol, propyl gallate, methyl p-hydroxybenzoate, ethyl p-hydroxybenzoate, butyl p-hydroxybenzoate, propyl p-hydroxybenzoate, 2-mercaptobenzimidazole and the like.

Examples of the tackifier include, but are not limited to, ester gum, glycerol, glycerol ester of hydrogenated rosin, petroleum resin, rosin, polybutene and the like.

Examples of the plasticizer include, but are not limited to, polybutene, glycerol, glycerin fatty acid ester and the like.

Examples of the flavor include, but are not limited to, dl-menthol, orange oil, peppermint oil, lemon oil, rose oil and the like.

Examples of the filler include, but are not limited to, titanium oxide, zinc oxide, acrylic acid starch 100 and the like.

Examples of the thickener include, but are not limited to, carboxymethylcellulose, carrageenan, pectin, poly-N-vinylacetamide, copolymer of N-vinylacetamide and sodium acrylate and the like.

The patches preparation of the present invention comprises a support, the aforementioned adhesive layer formed on one surface (one side) of the support, and a release liner as appropriate on the other surface free of contact with the support of the adhesive layer. When in use, the release liner is peeled off and the adhesive layer of the patches preparation is adhered to the skin, whereby transdermal administration is performed.

The support in the present invention is a sheet for forming the adhesive layer and/or the adhesive additive layer in the patches preparation. The support is not particularly limited as long as it is made of a material impermeable or hardly permeable to WT1 protein-derived cancer antigen peptide, and uninfluential or hardly influential on the release of WT1 protein-derived cancer antigen peptide, and may be stretchable or nonstretchable. Examples thereof include, but are not limited to, resin film such as ethylcellulose, nylon, poly(ethylene terephthalate) (PET), polyester, polypropylene and the like, and a combination thereof. In addition, nonwoven fabric such as PET and the like may be formed on one side of a support on which an adhesive layer is not formed. Furthermore, it may be a support with a single layer structure or a structure of a laminate of plural materials. The support may be colorless and transparent, may be colored white or flesh color and the like. The support colored white or flesh color and the like may have a surface of the support coated with a dye or may have a support containing dye, pigment or the like uniformly kneaded therein.

The support surface on which an adhesive layer is formed is preferably subjected to, for example, a surface treatment such as corona discharge treatment, plasma treatment, oxidation treatment, hairline processing, sand mat processing and the like.

The patches preparation of the present invention can be produced by a conventional method. For example, the preparation can be produced according to the section relating to the production of a plaster preparation described in "Manual for the development of transdermal formulation" Edited by Mitsuo Matsumoto (1985) and the like. In addition, for example, the preparation can be produced by using the apparatus, method and the like described in "Development of Apparatus for Producing Patches Preparation for Transdermal Treatment System (MEMBRANE, 32(2), 116-119 (2007))".

To be specific, in the production of the patches preparation, particularly tapes preparation, of the present invention, a general production method of adhesive tapes can be applied to form an adhesive layer. Representative example thereof is a solvent coating method. Besides this, a hot-melt coating method, an electron beam cured emulsion coating method and the like can be used.

When the adhesive layer is formed by a solvent coating method, for example, an adhesive layer mixture is prepared by mixing a WT1 protein-derived cancer antigen peptide, a mixture comprising an adhesive, and a formulation component such as a particular ether-type additive, a curing agent and the like, and an organic solvent, the mixture is applied onto one surface of a support or a release liner, the organic solvent is removed by drying, and a release liner or a support is adhered at a certain timing before or after drying. The thickness of the obtained adhesive layer is within the range of about 10 - about 400 µm, preferably about 20 - about 200 µm. However, the thickness of the adhesive layer is not limited to these ranges, and any thickness greater or smaller than these ranges is within the scope of the present invention.

When an adhesive additive layer is formed in the patches preparation, for example, the following production method can be mentioned, but the method is not limited thereto.

An adhesive layer mixture is prepared by mixing a WT1 protein-derived cancer antigen peptide, a mixture comprising adhesive, a formulation component such as a particular ether-type additive, a curing agent and the like, and an organic solvent. An adhesive additive layer mixture is prepared by mixing a solution containing an adhesive, formulation components such as a particular ether-type additive, a curing agent and the like, and an organic solvent. The adhesive additive layer mixture is applied on one surface of the support, and the organic solvent is removed by drying, whereby a patches preparation a is produced. In addition, the adhesive layer mixture is applied on one surface of a release liner, and the organic solvent is removed by drying, whereby a patches preparation b is produced. The adhesive additive layer of the patches preparation a and the adhesive layer of the patches preparation b are adhered to produce the patches preparation. The patches preparation a and the patches preparation b may be produced simultaneously or one of them may be produced earlier.

In another production method, an adhesive additive layer mixture is applied on one surface of a support, and the organic solvent is removed by drying, whereby a patches preparation a is produced. The adhesive layer mixture is applied on the patches additive layer side of the patches preparation a, the organic solvent is removed by drying, and a release liner is adhered at a certain timing before or after drying, whereby a patches preparation is produced.

Furthermore, in another production method, an adhesive layer mixture is applied on one surface of a release liner, and the organic solvent is removed by drying, whereby a patches preparation a is produced. The adhesive additive layer mixture is applied on the adhesive layer side of the patches preparation a, the organic solvent is removed by drying, and the release liner is adhered at a certain timing before or after drying, whereby a patches preparation is produced.

The thickness of the obtained adhesive layer and adhesive additive layer is each within the range of about 5 - about 200 µm, preferably about 10 - about 100 µm, from which each can be selected freely. However, the thickness of the adhesive layer is not limited to these ranges, and any thickness greater or smaller than these ranges is within the scope of the present invention.

The total of the thickness of the adhesive layer and the thickness of the adhesive additive layer is about 10 - about 400 µm, preferably about 20 - about 200 pm. The ratio of the thickness of the adhesive layer and the thickness of the adhesive additive layer (thickness of adhesive layer:thickness of adhesive additive layer) is preferably 1:10 - 10:1, more preferably 1:10 - 1:1.

The release liner to cover the surface of the adhesive layer is appropriately selected. Examples of the release liner include, but are not limited to, a release liner having a release layer having detachability on the surface thereof, such as a paper liner treated with a silicone resin and the like, a plastic film and the like.

The blending formulations of ointment, gel, cream, lotion, and microneedle preparation, which are other transdermal preparations, are briefly explained.

Ointment contains, in addition to a WT1 protein-derived cancer antigen peptide and a particular ether-type additive, at least higher fatty acid such as myristic acid, lauric acid, palmitic acid, stearic acid, linoleic acid and the like or an ester thereof, waxes such as whale wax and the like, surfactant such as polyoxyethylene alkyl ether, sucrose ester of fatty acid and the like, hydrocarbons such as hydrophilic petrolatum, Plastibase and the like. As a preparation formulation of the ointment, for example, higher fatty acid or an ester thereof 5 - 15 wt%, surfactant 1 - 10 wt%, WT1 protein-derived cancer antigen peptide 0.5 - 10 wt%, and the aforementioned ether-type additive 0.1 - 20 wt% are mixed at room temperature or under heating, waxes 4 - 10 wt% and hydrocarbon 50 - 80 wt% are added, and the mixture is heated or melted by heating, maintained at 50 - 100°C and, after all components turn into a transparent dissolved solution, uniformly blended in a homomixer. Thereafter, the solution is cooled to room temperature with stirring to give ointment.

Gel contains, in addition to a WT1 protein-derived cancer antigen peptide and a particular ether-type additive, at least lower alcohol such as ethanol and the like, water, gelling agent such as carboxyvinyl polymer and the like, and neutralizing agent such as triethanolamine and the like. As a preparation formulation of the gel, for example, water 55 wt% or below and gelling agent 0.5 - 5 wt% are added to allow for swelling. On the other hand, WT1 protein-derived cancer antigen peptide 0.5 - 10 wt% and the aforementioned ether-type additive 0.1 - 20 wt% are dissolved in a mixture of glycols not more than 40 wt% and lower alcohol not more than 60 wt%. They are mixed, and neutralizing agent is further added to adjust to pH 4 - 7, whereby gelling agent is obtained.

Cream contains, in addition to a WT1 protein-derived cancer antigen peptide and a particular ether-type additive, at least higher fatty acid ester such as myristic acid ester, oleic acid ester and the like, water, hydrocarbons such as liquid paraffin and the like, and emulsifier such as polyoxyethylene alkyl ethers and the like. As a preparation formulation of the cream, for example, appropriate amounts of the above-mentioned WT1 protein-derived cancer antigen peptide, the aforementioned ether-type additive, higher fatty acid ester, water, hydrocarbons and emulsifier are mixed and stirred, whereby cream is obtained.

Lotion contains, in addition to a WT1 protein-derived cancer antigen peptide and a particular ether-type additive, at least lower alcohol such as ethanol and the like, water, glycerol, and/or glycols as a substrate. As a preparation formulation of the lotion, for example, appropriate amounts of the above-mentioned WT1 protein-derived cancer antigen peptide, the aforementioned ether-type additive, lower alcohol, water, and/or glycols are mixed and stirred, whereby lotion is obtained.

As the material of the microneedle preparation, the same metals as those for conventional injections, silicon, biodegradable polymer, glass and the like can be mentioned. Microneedles of a solid type directly coated on the outside of the needle, a type having a hollow structure like the injection needle, a self-dissolving type using a substance soluble in the body as a base and the like have been developed. It is obtained by forming any type of these microneedles by using them together with a WT1 protein-derived cancer antigen peptide and a particular ether-type additive.

The transdermal preparation of the present invention can contain, as in the case of a patches preparation, pharmaceutically acceptable conventional formulation components such as stabilizer, flavor, filler, thickener and the like, as long as the object of the present invention is not impaired.

The transdermal preparation of the present invention further comprises lactic acid. Lactic acid is added to the transdermal preparation of the present invention in combination with the below-mentioned "WT1 protein-derived cancer antigen 54 peptide degradation inhibitor".

The "WT1 protein-derived cancer antigen peptide degradation inhibitor" that is added to the transdermal preparation of the present invention is a degradation inhibitor of WT1 protein-derived cancer antigen peptide during co-culture to living skin-derived components. To be specific, when WT1 protein-derived cancer antigen peptide is added to an enzyme fraction extracted from a mouse skin homogenate, the peptide is degraded. It is a substance that suppresses degradation of the peptide when it is co-present.

Specifically, a substance that suppresses degradation of the peptide can be found by performing the test shown below.

400 µl of a 30 µg/ml solution of hairless mouse· homogenate is added into a 1.5 ml polypropylene tube, water or a degradation suppressive substance solution is added by 50 µl, and 200 µg/ml solution of the peptide of SEQ ID NO: 2 in water is added by 50 µl to the total amount of 500 µl. The solution is incubated at 37°C for 1 hr, and the enzyme reaction was stopped by adding 500µl of 80% ethanol. Thereafter, centrifugation is performed, the concentration of the peptide of SEQ ID NO: 2 in the supernatant is quantified by the high performance liquid chromatography method, and the degradation inhibition rate is calculated from the amount of degraded peptide.

In the above-mentioned test, a peptide having the amino acid sequence shown in SEQ ID NO: 2 is used. A degradation inhibitor can be appropriately found by performing a similar test for other WT1 protein-derived cancer antigen peptides.

The WT1 protein-derived cancer antigen peptide degradation inhibitor is selected from the group consisting of decanoic acid, sodium deoxycholate, and N-lauroylsarcosine.

While the rate of WT1 protein-derived cancer antigen peptide and particular ether-type additive to be contained in the transdermal preparation of the present invention is optional, it is generally 0.1 - 40 wt% and 0.01 - 50 wt%, preferably 0.5 - 20 wt% and 0.1 - 30 wt%, more preferably 0.5 - 15 wt% and 0.5 - 20 wt%, further preferably 0.5 - 10 wt% and 0.5 - 15 wt%, most preferably 1 - 10 wt% and 1 - 15 wt%, respectively, in the transdermal preparation as 100 wt%.

When the present invention is a patches preparation, the rate of WT1 protein-derived cancer antigen peptide and particular ether-type additive to be contained in the adhesive layer is optional. It is generally 0.1 - 40 wt% and 0.01 - 50 wt%, preferably 0.5 - 20 wt% and 0.1 - 30 wt%, more preferably 0.5 - 15 wt% and 0.5 - 20 wt%, further preferably 0.5 - 10 wt% and 0.5 - 15 wt%, most preferably 1 - 10 wt% and 1 - 15 wt%, respectively, in the adhesive layer as 100 wt%.

The CTL induction activity of the transdermal preparation of the present invention can be detected by measuring the number of CTL by the HLA tetramer method (Int. J. Cancer: 100, 565-570 (2002)) or limiting dilution method (Nat. Med.: 4, 321-327 (1998)). Alternatively, for example, HLA-A24-restricted CTL induction activity can be confirmed by tests using the HLA-A*0201 transgenic mouse described in Experimental Examples 1 - 5, and HLA-A24-restricted CTL induction activity can be confirmed by tests using the HLA-A24* model mouse described in WO 02/47474.

The transdermal preparation of the present invention has specific CTL induction capacity according to the WT1 protein-derived cancer antigen peptide to be contained, and the induced CTL shows an anticancer action via a cytotoxic action and lymphokine production. Therefore, the transdermal preparation of the present invention can be used as a cancer vaccine for the treatment or prophylaxis of cancer.

### Examples

The present invention is explained in more detail in the following by referring to Examples, Reference Examples, Experimental Examples and the like, which are not to be construed as limitative. In the following Examples and the like, "%" means "wt%".

As a support, 50.8 µm poly(ethylene terephthalate), and/or ethylene vinyl acetate copolymer laminate film (Scotchpak #9732), manufactured by 3M Health Care Limited, were/was used. As a release liner, Bynasheet 64S-018B manufactured by FUJIMORI KOGYO CO., LTD. was used.

### Example 1 (not according to the invention)

Acrylic adhesive (DURO-TAK 387-2287, manufactured by Henkel, solid content 51 wt%, 0.794 g), ethyl acetate (0.2 mL), and 10% of α-monoisostearyl glyceryl ether in the adhesive layer were mixed. To the mixture was added the peptide of SEQ ID NO: 2, which was dissolved in methanol (0.5 ml), such that its content percentage in the adhesive layer was 9%, and the mixture was stirred well. The obtained mixture was spread on a support such that the thickness of the adhesive layer after drying was about 60 µm, and the layer was dried at room temperature for one day. Then, a release liner was adhered thereto to give tapes preparation 1.

### Examples 2 - 5 (not according to the invention)

Using the additives shown in the following Table 1 instead of α-monoisostearyl glyceryl ether in Example 1, and in the same manner as in Example 1, tapes preparations 2 - 5 were produced.

**Table 1**

| | additive |
|---|---|
| Example 2 =tapes preparation 2 | monooleyl glyceryl ether |
| Example 3 =tapes preparation 3 | polyoxyethylene isostearyl ether (average mole number of added ethylene oxide: 5) |
| Example 4 =tapes preparation 4 | polyoxyethylene oleyl ether (average mole number of added ethylene oxide: 2) |
| Example 5 =tapes preparation 5 | polyoxyethylene alkyl (12 - 14) ether (average mole number of added ethylene oxide: 3) |

### Reference Examples 1 - 5

Using the additives shown in the following Table instead of α-monoisostearyl glyceryl ether in Example 1, and in the same manner as in Example 1, tapes preparations A - E were produced.

**Table 2**

| | additive |
|---|---|
| Reference Example 1 =tapes preparation A | lactic acid |
| Reference Example 2 =tapes preparation B | isostearyl glyceryl ester |
| Reference Example 3 =tapes preparation C | isostearyl alcohol |
| Reference Example 4 =tapes preparation D | oleyl glyceryl ester |
| Reference Example 5 =tapes preparation E | polyoxyethylene polyoxypropylene cetyl ether (average mole number of added ethylene oxide: 1) |

### Example 6

Acrylic adhesive (DURO-TAK 387-2287, manufactured by Henkel, solid content 51 wt%, 0.598 g), ethyl acetate (0.2 mL), and 10% of α-monoisostearyl glyceryl ether, lactic acid and N-lauroylsarcosine, respectively, in the adhesive layer were mixed. To the mixture was added the peptide of SEQ ID NO: 2, which was dissolved in methanol (0.5 ml), such that its content percentage in the adhesive layer was 9%, and the mixture was stirred well. The obtained mixture was spread on a support such that the thickness of the adhesive layer after drying was about 60 µm, and the layer was dried at room temperature for one day. Then, a release liner was adhered thereto to give tapes preparation 6.

### Example 7

Using sodium deoxycholate instead of N-lauroylsarcosine in Example 6, and in the same manner as in Example 6, tapes preparation 7 was produced.

### Example 8

Using decanoic acid instead of N-lauroylsarcosine in Example 6, and in the same manner as in Example 6, tapes preparation 8 was produced.

### Example 9 (not according to the invention)

Acrylic adhesive (DURO-TAK 387-2287, manufactured by Henkel, solid content 51 wt%, 0.843 g), ethyl acetate (0.2 mL), and 5% of α-monoisostearyl glyceryl ether in the adhesive layer were mixed. To the mixture was added the peptide of SEQ ID NO: 2, which was dissolved in methanol (0.5 ml), such that its content percentage in the adhesive layer was 9%, and the mixture was stirred well. The obtained mixture was spread on a support such that the thickness of the adhesive layer after drying was about 60 µm, and the layer was dried at room temperature for one day. Then, a release liner was adhered thereto to give tapes preparation 9.

### Example 10 (not according to the invention)

Acrylic adhesive (DURO-TAK 387-2287, manufactured by Henkel, solid content 51 wt%, 0.794 g), ethyl acetate (0.2 mL), and 5% of α-monoisostearyl glyceryl ether and polyoxyethylene isostearyl ether (average mole number of added ethylene oxide: 5), respectively, in the adhesive layer were mixed. To the mixture was added the peptide of SEQ ID NO: 2, which was dissolved in methanol (0.5 ml), such that its content percentage in the adhesive layer was 9%, and the mixture was stirred well. The obtained mixture was spread on a support such that the thickness of the adhesive layer after drying was about 60 µm, and the layer was dried at room temperature for one day. Then, a release liner was adhered thereto to give tapes preparation 10.

### Example 11

Acrylic adhesive (DURO-TAK 387-2287, manufactured by Henkel, solid content 51 wt%, 0.677 g), ethyl acetate (0.2 mL), and 10% of α-monoisostearyl glyceryl ether, lactic acid and N-lauroylsarcosine, respectively, in the adhesive layer were mixed. To the mixture was added the peptide of SEQ ID NO: 2, which was dissolved in methanol (0.5 ml), such that its content percentage in the adhesive layer was 1%, and the mixture was stirred well. The obtained mixture was spread on a support such that the thickness of the adhesive layer after drying was about 60 µm, and the layer was dried at room temperature for one day. Then, a release liner was adhered thereto to give tapes preparation 11.

### Example 12

Acrylic adhesive (DURO-TAK 387-2287, manufactured by Henkel, solid content 51 wt%, 0.686 g), ethyl acetate (0.3 mL), and 1%, 10% and 10% of α-monoisostearyl glyceryl ether, lactic acid and decanoic acid, respectively, in the adhesive layer were mixed. To the mixture was added the peptide of SEQ ID NO: 2, which was dissolved in methanol (0.4 ml), such that its content percentage in the adhesive layer was 9%, and the mixture was stirred well. The obtained mixture was spread on a support such that the thickness of the adhesive layer after drying was about 60 µm, and the layer was dried at room temperature for one day. Then, a release liner was adhered thereto to give a tapes preparation 12.

### Example 13

Acrylic adhesive (DURO-TAK 387-2287, manufactured by Henkel, solid content 51 wt%, 0.549 g), ethyl acetate (0.3 mL), and 15%, 10% and 10% of α-monoisostearyl glyceryl ether, lactic acid and decanoic acid, respectively, in the adhesive layer were mixed. To the mixture was added the peptide of SEQ ID NO: 2, which was dissolved in methanol (0.4 ml), such that its content percentage in the adhesive layer was 9%, and the mixture was stirred well. The obtained mixture was spread on a support such that the thickness of the adhesive layer after drying was about 60 µm, and the layer was dried at room temperature for one day. Then, a release liner was adhered thereto to give tapes preparation 13.

### Example 14

Acrylic adhesive (DURO-TAK 387-2287, manufactured by Henkel, solid content 51 wt%, 0.598 g), ethyl acetate (0.3 mL), and 10% of polyoxyethylene isostearyl ether (average mole number of added ethylene oxide: 15), lactic acid and decanoic acid, respectively, in the adhesive layer were mixed. To the mixture was added the peptide of SEQ ID NO: 2, which was dissolved in methanol (0.4 ml), such that its content percentage in the adhesive layer was 9%, and the mixture was stirred well. The obtained mixture was spread on a support such that the thickness of the adhesive layer after drying was about 60 µm, and the layer was dried at room temperature for one day. Then, a release liner was adhered thereto to give tapes preparation 14.

### Example 15

Using polyoxyethylene 2-ethylhexyl ether (average mole number of added ethylene oxide: 4) instead of polyoxyethylene isostearyl ether (average mole number of added ethylene oxide: 15) in Example 14, and in the same manner as in Example 14, tapes preparation 15 was produced.

### Example 16 (not according to the invention)

Acrylic adhesive (DURO-TAK 387-2287, manufactured by Henkel, solid content 51 wt%, 0.794 g), ethyl acetate (0.3 mL), and 10% of α-monoisostearyl glyceryl ether in the adhesive layer were mixed. To the mixture was added the peptide of SEQ ID NO: 2, which was dissolved in methanol (0.4 ml), such that its content percentage in the adhesive layer was 9%, and the mixture was stirred well. The obtained mixture was spread on a release liner such that the thickness of the adhesive layer after drying was about 30 µm, and the layer was dried at room temperature for one day to give tapes preparation 16a. In addition, acrylic adhesive (DURO-TAK 387-2287, manufactured by Henkel, solid content 51 wt%, 0.882 g), ethyl acetate (0.7 mL), and 10% of α-monoisostearyl glyceryl ether in the adhesive additive layer were mixed. The obtained mixture was spread on a support such that the thickness of the adhesive additive layer after drying was about 30 µm, and the layer was dried at room temperature for one day to give tapes preparation 16b. Then, the tapes preparation 16a and the tapes preparation 16b were adhered to each other to give tapes preparation 16.

### Example 17

Acrylic adhesive (DURO-TAK 387-2287, manufactured by Henkel, solid content 51 wt%, 0.598 g), ethyl acetate (0.2 mL), and 10% of α-monoisostearyl glyceryl ether, lactic acid and N-lauroylsarcosine, respectively, in the adhesive layer were mixed. To the mixture was added the peptide of SEQ ID NO: 2, which was dissolved in methanol (0.5 ml), such that its content percentage in the adhesive layer was 9%, and the mixture was stirred well. The obtained mixture was spread on a release liner such that the thickness of the adhesive layer after drying was about 30 µm, and the layer was dried at room temperature for one day. Then, a release liner was adhered thereto to give tapes preparation 17a. In addition, acrylic adhesive (DURO-TAK 387-2287, manufactured by Henkel, solid content 51 wt%, 0.686 g), ethyl acetate (0.7 mL), and 10% of α-monoisostearyl glyceryl ether, lactic acid and N-lauroylsarcosine, respectively, in the adhesive additive layer were mixed. The obtained mixture was spread on a support such that the thickness of the adhesive additive layer after drying was about 30 µm, and the layer was dried at room temperature for one day to give tapes preparation 17b. Then, the tapes preparation 17a and the tapes preparation 17b were adhered to each other to give tapes preparation 17.

### Example 18

Acrylic adhesive (DURO-TAK 387-2287, manufactured by Henkel, solid content 51 wt%, 0.598 g), ethyl acetate (0.3 mL), and 10% of α-monoisostearyl glyceryl ether, lactic acid and decanoic acid, respectively, in the adhesive layer were mixed. To the mixture was added the peptide of SEQ ID NO: 2, which was dissolved in methanol (0.4 ml), such that its content percentage in the adhesive layer was 9%, and the mixture was stirred well. The obtained mixture was spread on a release liner such that the thickness of the adhesive layer after drying was about 10 µm, and the layer was dried at room temperature for one day. Then, a release liner was adhered thereto to give tapes preparation 18a. In addition, acrylic adhesive (DURO-TAK 387-2287, manufactured by Henkel, solid content 51 wt%, 0.686 g), ethyl acetate (0.7 mL), and 10% of α-monoisostearyl glyceryl ether, lactic acid and decanoic acid, respectively, in the adhesive additive layer were mixed. The obtained mixture was spread on a support such that the thickness of the adhesive additive layer after drying was about 50 µm, and the layer was dried at room temperature for one day to give tapes preparation 18b. Then, the tapes preparation 18a and the tapes preparation 18b were adhered to each other to give tapes preparation 18.

### Experimental Example 1 (not according to the invention)

### <CTL induction by tapes preparation containing peptide of SEQ ID NO: 2 (1) >

The specific CTL induction capacity of tapes preparation 1 produced in Example 1 was evaluated using HLA-A*0201 transgenic mouse (Eur. J. Immunol.:34, 3060, 2004).

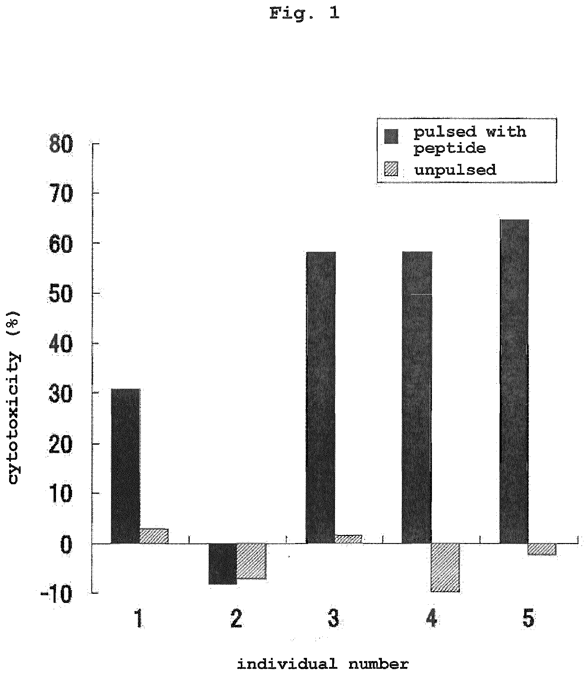

The tapes preparation (3 cm², peptide dose 1.5 mg) was adhered to the back of each of five HLA-A*0201 transgenic mice. At 3 days after adhesion, the tapes preparation was detached. At 7 days after the start of adhesion (4 days after detachment), the spleen was isolated, and splenocytes were prepared. A part of the splenocytes was pulsed with 100 µmol/l administered peptide for 1 hr. Pulsing means addition of peptide to splenocytes to allow for binding of antigen peptide to HLA on the cellular surface. Splenocytes not pulsed with peptide and the above-mentioned splenocytes pulsed with peptide were mixed, plated on 24 well plate at 7.8×10⁶ cells/well and cultured. The culture medium was RPMI1640 medium supplemented with 10% FCS, 10 mmol/l HEPES, 2 mmol/l L-glutamine, 1 mM sodium pyruvate, 0.1 mmol/l MEM non-essential amino acid, 1% MEM vitamin, and 55 µmol/l 2-mercaptoethanol, and the cells were incubated for 6 days. The activity of CTL specific to the administered peptide in the cultured splenocytes was measured by ⁵¹Cr-release assay (J. Immunol.:159, 4753, 1997). As the target cell, EL4-HHD cell which is a cell line produced by gene transfer into mouse lymphoma-derived cell line in Pasteur Institute (J. Exp. Med.:185, 2043, 1997) was used for stable expression of MHC class I molecule of HLA-A*0201 and H-2D^{b} chimera. The target cell (3.7 MBq/5×10⁵ cells) was labeled with ⁵¹Cr, the aforementioned peptide was added at 167 µmol/l and the cell was further pulsed for 1 hr. The target cell not pulsed with peptide (unpulsed) was labeled with ⁵¹Cr for 2 hr and used as control target cell. The labeled target cells and splenocytes prepared previously were mixed at a ratio of 1:80, incubated for 5 hr, and the cytotoxicity of CTL was determined from the proportion of injured target cells. The results are shown in Fig. 1.

As shown in Fig. 1, the splenocyte prepared from the spleen of the mouse administered with tapes preparation 1 of the present invention strongly injured the target cell pulsed with peptide, but was weakly injurious to the control target cell not pulsed with the peptide, which clarified that peptide-specific CTL was induced. Therefrom it is clear that the tapes preparation of the present invention activates induction of CTL in vivo.

### Experimental Example 2 (not according to the invention)

### <CTL induction by tapes preparation containing peptide of SEQ ID NO: 2 (2) >

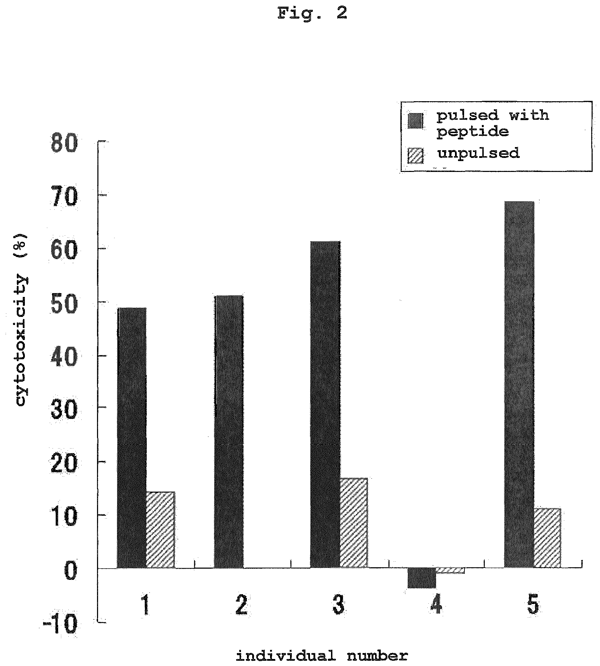

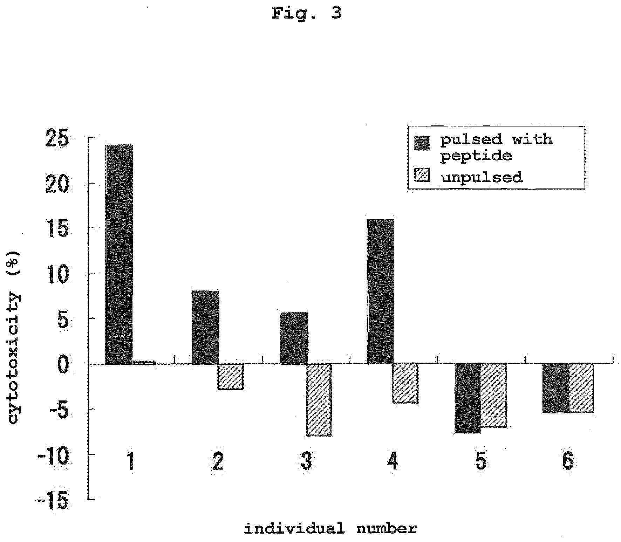

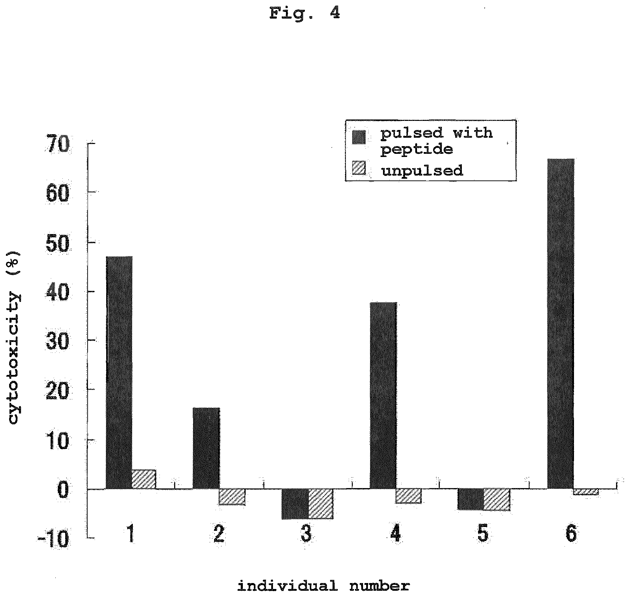

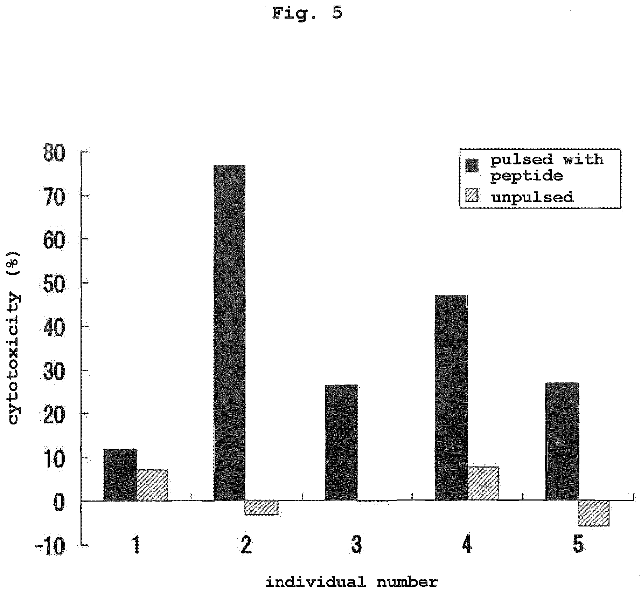

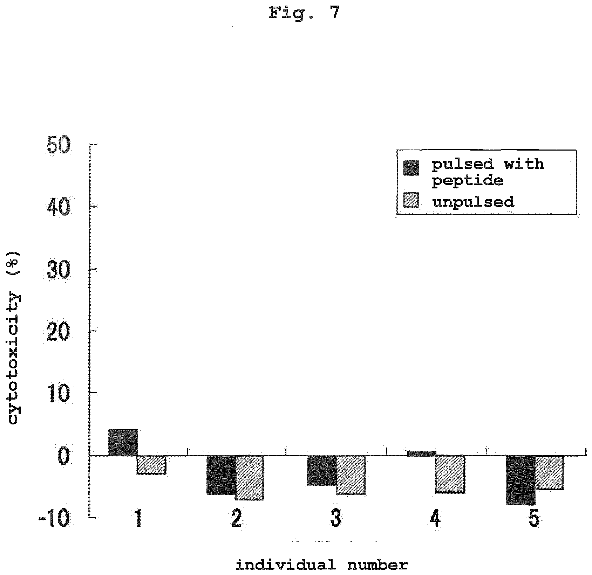

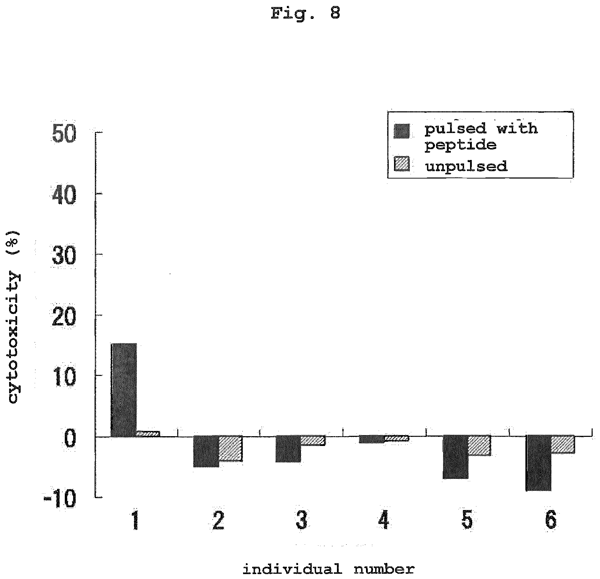

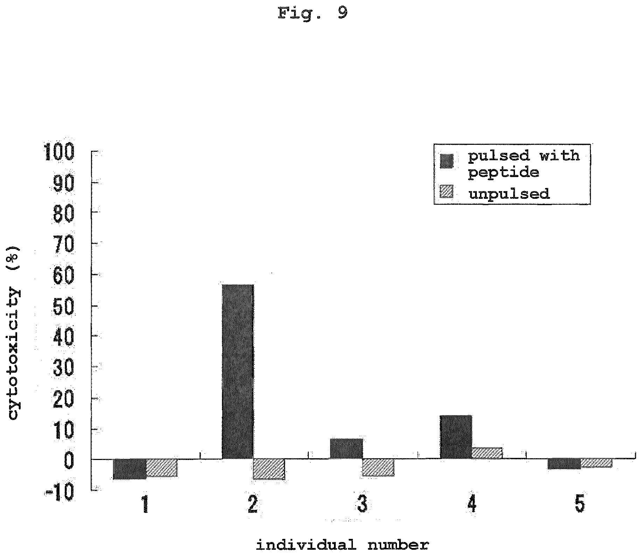

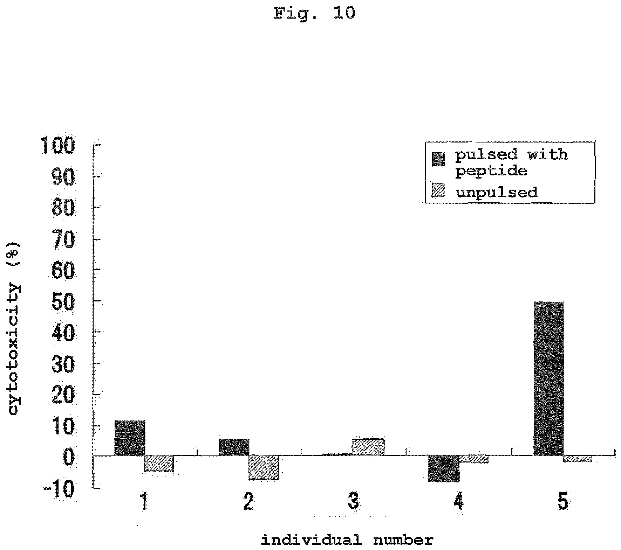

The specific CTL induction capacity of tapes preparations 2 - 5 and A - E produced in Examples 2 - 5 and Reference Examples 1 - 5 was evaluated in the same manner as in Experimental Example 1. One tapes preparation was administered to 5 or 6 HLA-A*0201 transgenic mice. ⁵¹Cr-release assay target cells and splenocyte were mixed at a ratio of 1:80 or 1:160 and incubated for 4 - 6 hr, and the cytotoxicity of CTL was determined from the proportion of injured target cells. The results are shown in Fig. 2 - Fig. 10.

As shown in Fig. 2 - Fig. 5, the splenocyte prepared from the spleen of the mouse administered with tapes preparations 2 - 5 of the present invention produced in Examples 2 - 5 strongly injured the target cell pulsed with peptide, but was weakly injurious to the control target cell not pulsed with the peptide, which clarified that peptide-specific CTL was induced.

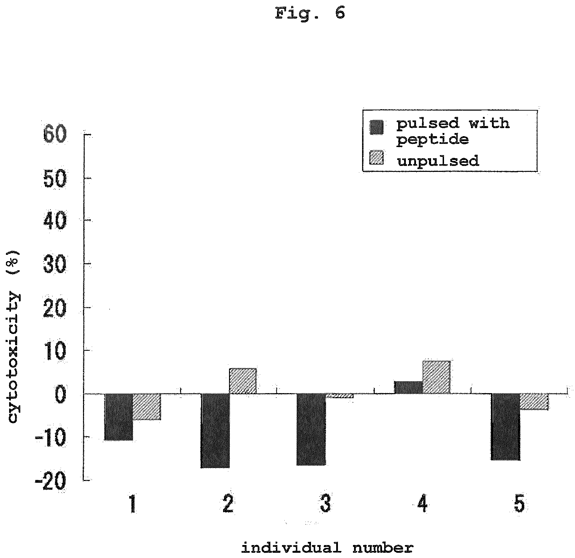

On the other hand, as shown in Fig. 6 - Fig. 10, the splenocyte prepared from the spleen of the mouse administered with tapes preparations A - E produced in Reference Examples 1 - 5 was weakly injurious to the target cell pulsed with peptide, which clarified that the tapes preparations A - E do not have an activity to induce peptide-specific CTL.

From such results and the results of Experimental Example 1, it was clarified that tapes preparation A added with lactic acid instead of the ether-type additive of the present invention does not have CTL induction activity in vivo. In addition, it was clarified that tapes preparations B and D added with ester such as isostearyl glyceryl ester and oleyl glyceryl ester instead of the ether-type additive of the present invention and tapes preparation C added with alcohol such as isostearyl alcohol were clarified to have no CTL induction activity in vivo. Furthermore, tapes preparation E added with polyoxyethylene polyoxypropylene cetyl ether having different structural characteristics instead of the ether-type additive of the present invention was clarified to have no CTL induction activity in vivo.

### Experimental Example 3

### <CTL induction by tapes preparation containing peptide of SEQ ID NO: 2 (3)>

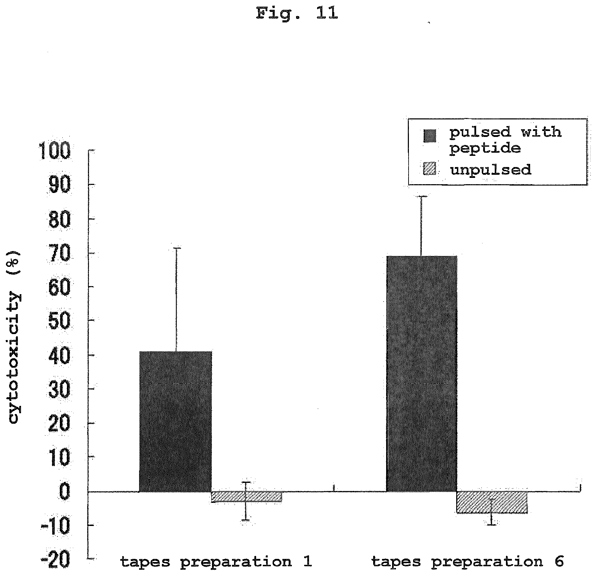

The specific CTL induction capacity of tapes preparations 6 - 8 produced in Examples 6 - 8 was evaluated in the same manner as in Experimental Example 1. One tapes preparation was administered to 4 or 5 HLA-A*0201 transgenic mice. ⁵¹Cr-release assay target cells and splenocyte were mixed at a ratio of 1:80 and incubated for 4 - 6 hr, and the cytotoxicity of CTL was determined from the proportion of injured target cells. The results are shown in Fig. 11 and Fig. 12. The cytotoxicity is shown by an average of 4 or 5 mice of each administration group.

As shown in Fig. 11, Fig. 12, the splenocyte prepared from the spleen of the mouse administered with tapes preparations 6 - 8 of the present invention strongly injured the target cell pulsed with peptide, but was weakly injurious to the control target cell not pulsed with the peptide, which clarified that peptide-specific CTL was induced. Furthermore, from the comparison of CTL induction activity of tapes preparation 1 and CTL induction activity of tapes preparations 6 - 8, it was clarified that the addition of lactic acid and N-lauroylsarcosine, lactic acid and sodium deoxycholate, or lactic acid and decanoic acid, in addition to α-monoisostearyl glyceryl ether, potentiates peptide-specific CTL induction activity.

### Experimental Example 4 (not according to the invention)

### <CTL induction by tapes preparation containing peptide of SEQ ID NO: 2 (4) >

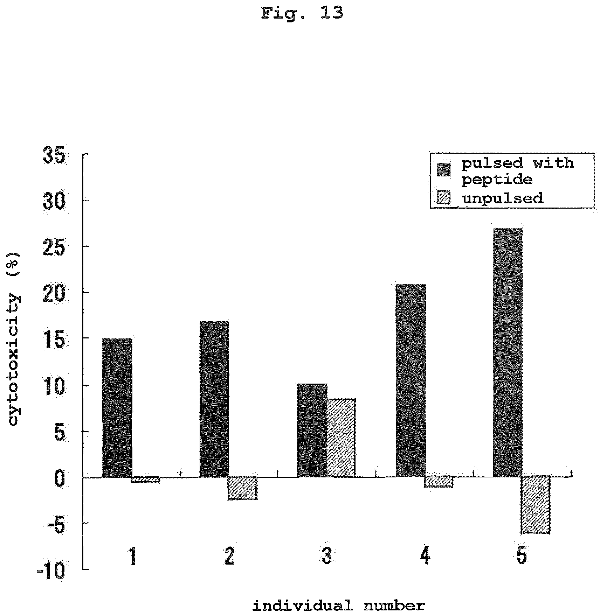

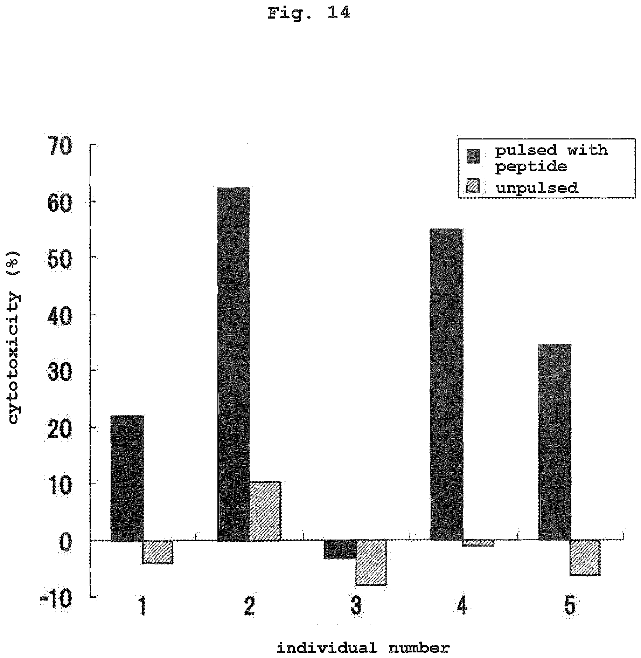

The specific CTL induction capacity of tapes preparations 9, 10 produced in Examples 9, 10 was evaluated in the same manner as in Experimental Example 1. One tapes preparation was administered to 5 HLA-A*0201 transgenic mice. ⁵¹Cr-release assay target cells and splenocyte were mixed at a ratio of 1:160 and incubated for 5 - 6 hr, and the cytotoxicity of CTL was determined from the proportion of injured target cells. The results are shown in Fig. 13 and Fig. 14.

As shown in Fig. 13, Fig. 14, the splenocyte prepared from the spleen of the mouse administered with tapes preparations 9, 10 of the present invention strongly injured the target cell pulsed with peptide, but was weakly injurious to the control target cell not pulsed with the peptide, which clarified that peptide-specific CTL was induced.

From such results, it was clarified that the tapes preparation wherein the amount of the ether-type additive of the present invention to be added was reduced to 5%, and the tapes preparation wherein the ether-type additives were combined also have CTL induction activity in vivo.

### Experimental Example 5

### <CTL induction by tapes preparation containing peptide of SEQ ID NO: 2 (5) >

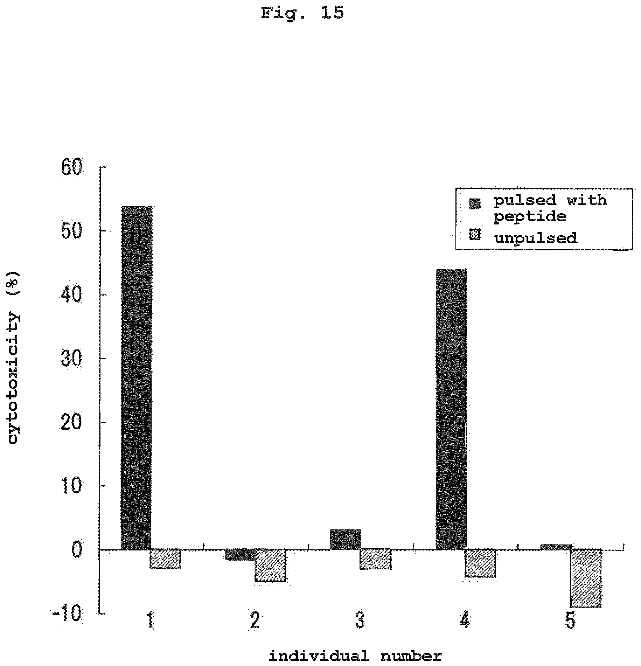

The specific CTL induction capacity of tapes preparation 11 produced in Examples 11 was evaluated in the same manner as in Experimental Example 1. The tapes preparation (3 cm², peptide dose 0.17 mg) was adhered to the back of each of five HLA-A*0201 transgenic mice. ⁵¹Cr-release assay target cells and splenocyte were mixed at a ratio of 1:80 and incubated for 5 - 6 hr, and the cytotoxicity of CTL was determined from the proportion of injured target cells. The results are shown in Fig. 15.

As shown in Fig. 15, the splenocyte prepared from the spleen of the mouse administered with tapes preparation 11 of the present invention strongly injured the target cell pulsed with peptide, but was weakly injurious to the control target cell not pulsed with the peptide, which clarified that peptide-specific CTL was induced.

From such results, it was clarified that the tapes preparation wherein the amount of peptide in the present invention was reduced to 1% also has CTL induction activity in vivo.

### Experimental Example 6:

### <CTL induction by tapes preparation containing peptide of SEQ ID NO: 2 (6)>

The specific CTL induction capacity of tapes preparations 12, 13 produced in Examples 12, 13 was evaluated using HLA-A*0201 transgenic mouse.

The tapes preparation (3 cm²) was adhered to the back of each of five HLA-A*0201 transgenic mice. At 3 days after adhesion, the tapes preparation was detached. At 7 days after the start of adhesion (4 days after detachment), the spleen was isolated, and splenocytes were prepared. IFNγ ELISPOT assay kit was used for the measurement of IFNγ production. On the previous day of splenocyte preparation, an ELISPOT plate was treated with an anti-IFNγ antibody, and blocked with RPMI1640 medium containing 10% FBS the next day. The prepared splenocytes were plated on the blocked ELISPOT plate at 1.0×10⁶ cells/well. The peptide of SEQ ID NO: 2 was dissolved in DMSO at 40 mg/mL, and further diluted with 10% FBS-containing RPMI1640 medium to 20 µg/mL. The diluted peptide was added at 50 µL/well to splenocytes derived from an animal administered with the peptide. The splenocytes added with the peptide were incubated for 15 hr at 37°C in a 5% CO₂ incubator, whereby peptide re-stimulation in vitro was performed. After culture, the supernatant was removed, and biotinylated IFNγ antibody was added to the ELISPOT plate. Avidinated HRP was added, and HRP substrate was added to cause color development. The number of the spot that developed color was measured by ImmunoSpot.

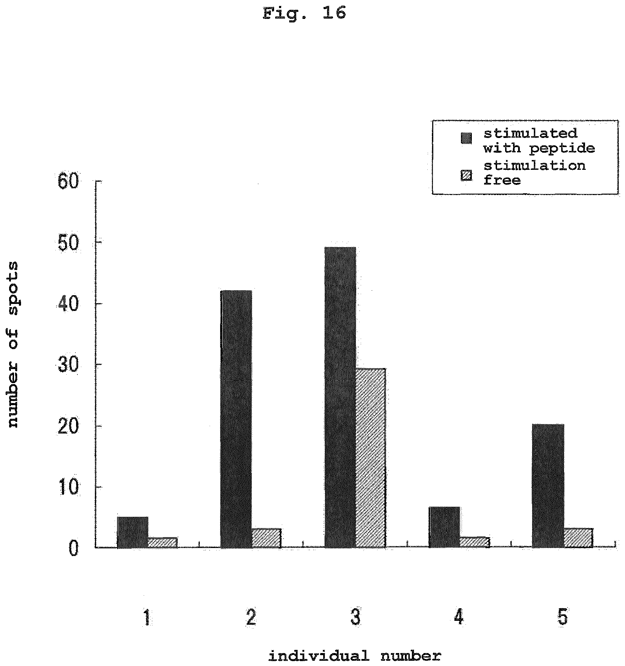

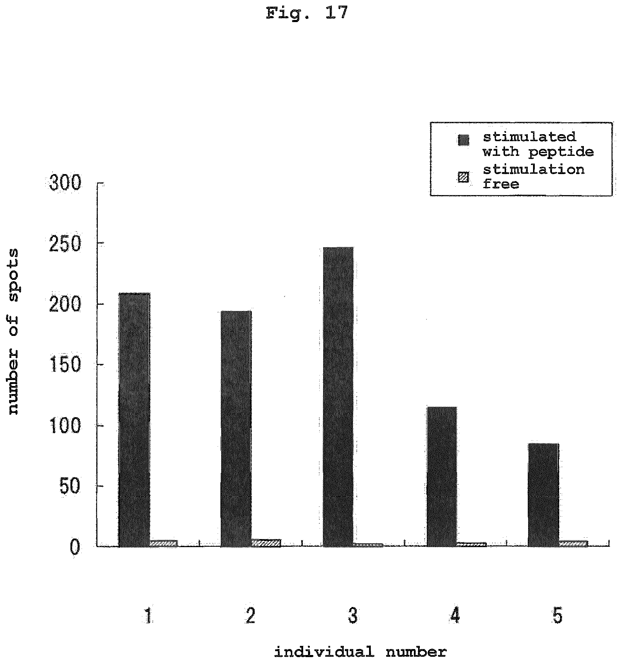

The results of IFNγ ELISPOT assay are shown in Fig. 16, Fig. 17.

As shown in Fig. 16, Fig. 17, peptide-specific IFNγ production was confirmed in the splenocytes derived from HLA-A*0201 transgenic mouse, from which the tapes preparations of Examples 12 and 13 were found to have CTL induction capacity.

From such results, it was clarified that the tapes preparation wherein the amount of the ether-type additive of the present invention was reduced to 1%, and the tapes preparation wherein the amount to be added was increased to 15% also have CTL induction activity in vivo.

### Experimental Example 7:

### <CTL induction by tapes preparation containing peptide of SEQ ID NO: 2 (7) >

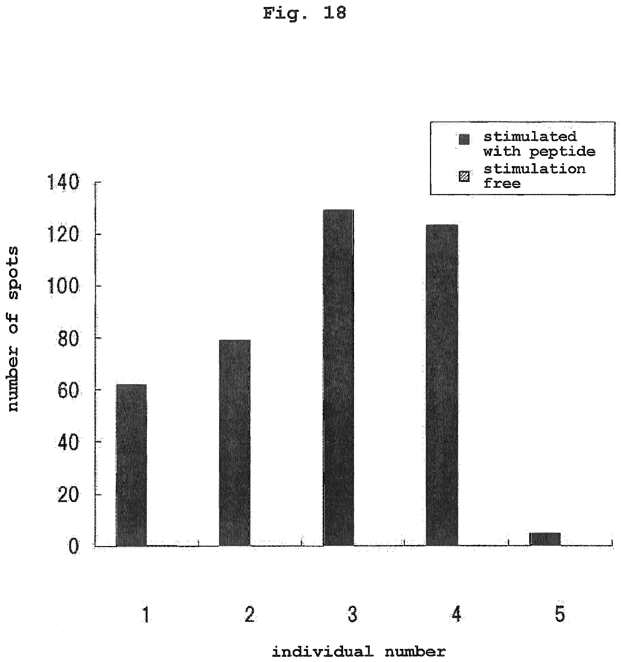

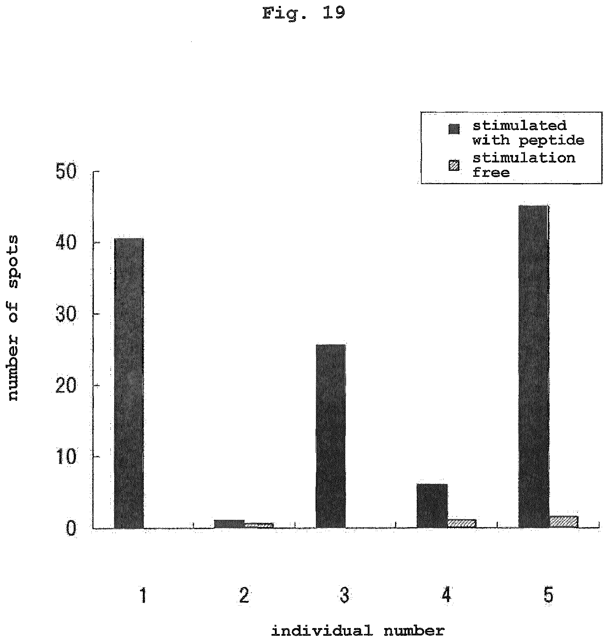

The specific CTL induction capacity of tapes preparations 14, 15 produced in Examples 14, 15 was evaluated in the same manner as in Experimental Example 6. One tapes preparation was administered to 5 HLA-A*0201 transgenic mice. The prepared splenocytes were plated on the blocked ELISPOT plate at 0.5×10⁶ cells/well, measured by ImmunoSpot, and the CTL induction capacity was determined. The results are shown in Fig. 18, Fig. 19.

As shown in Fig. 18, Fig. 19, peptide-specific IFNγ production was confirmed in the splenocytes derived from HLA-A*0201 transgenic mouse, from which the tapes preparations of Examples 14 and 15 were found to have CTL induction capacity.

From such results, it was clarified that the tapes preparation using polyoxyethylene isostearyl ether having a mole number of added polyethylene of 15 of the polyoxyethylene(POE)ether-type additive of the present invention, and the tapes preparation using polyoxyethylene 2-ethylhexylether containing a hydrocarbon group having 8 carbon atoms also have CTL induction activity in vivo.

### Experimental Example 8:

### <CTL induction by tapes preparation containing peptide of SEQ ID NO: 2 (8) >

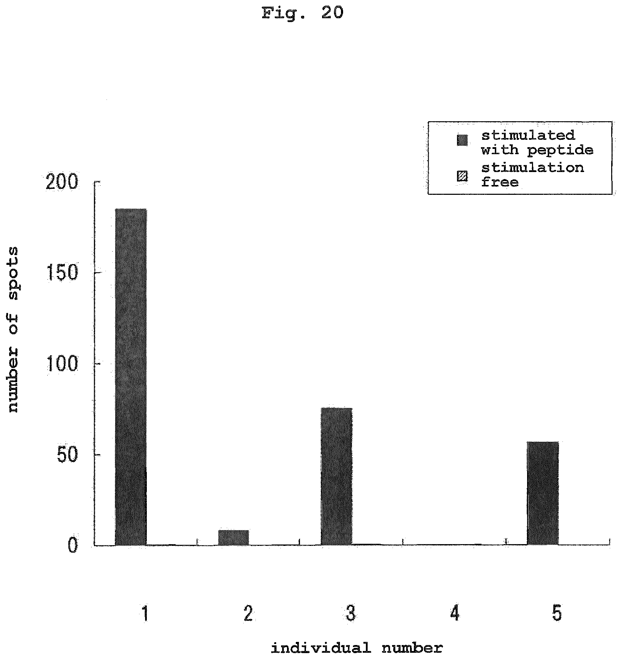

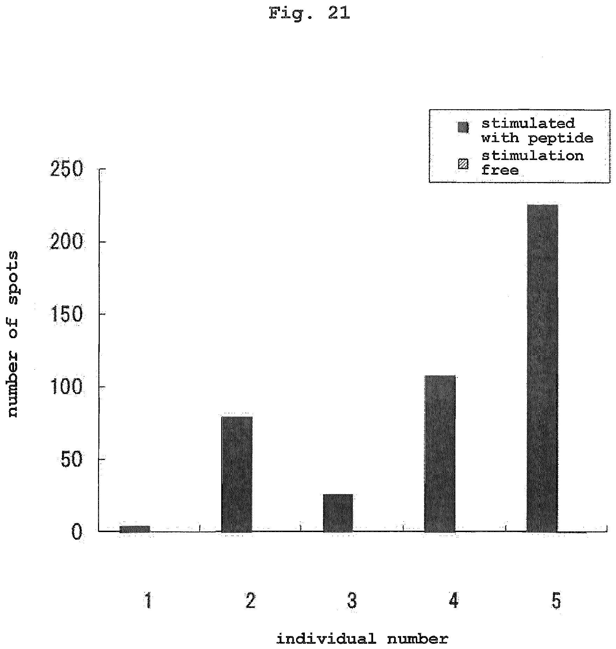

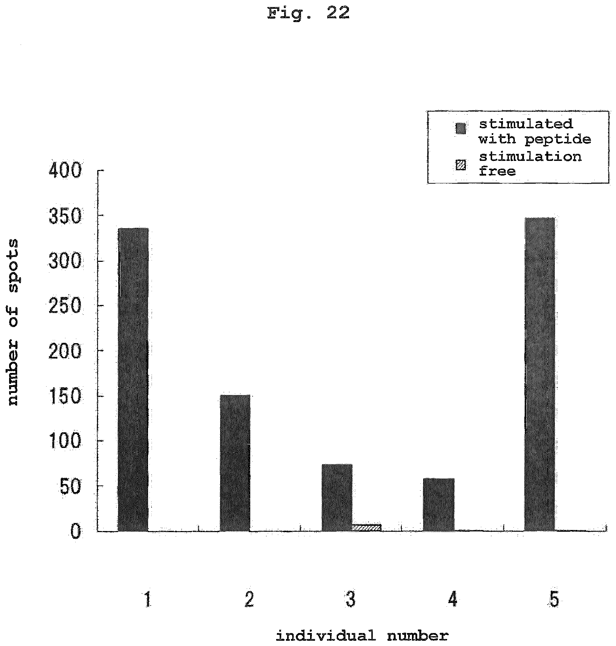

The specific CTL induction capacity of tapes preparations 16 - 18 produced in Examples 16 - 18 was evaluated in the same manner as in Experimental Example 6. One tapes preparation was administered to 5 HLA-A*0201 transgenic mice. The prepared splenocytes were plated on the blocked ELISPOT plate at 0.5×10⁶ cells/well, measured by ImmunoSpot, and the CTL induction capacity was determined. The results are shown in Fig. 20 - Fig. 22.

As shown in Fig. 20 - Fig. 22., peptide-specific IFNγ production was confirmed in the splenocytes derived from HLA-A*0201 transgenic mouse, from which the tapes preparations of Examples 16 - 18 were found to have CTL induction capacity.

From such results, it was clarified that the tapes preparation of the present invention, even when containing a peptide-containing adhesive layer having a thickness of 1/2 or 1/6, shows almost equivalent CTL induction activity as long as the total thickness of the adhesive layer and the adhesive additive layer is equivalent.

### Industrial Applicability

The transdermal preparation of the present invention is a cancer vaccine preparation that enables transdermal administration of WT1 protein-derived cancer antigen peptide without invasiveness due to the injection preparation, and maintenance of the drug concentration in the vicinity of the administration site for a longer time in a sustained manner. The administration using the transdermal preparation of the present invention also facilitates confirmation and discontinuation of medication.

This application is based on patent application No. 2012-148639 filed in Japan (filing date: July 2, 2012).

## Claims

1. A transdermal preparation comprising a WT1 protein-derived cancer antigen peptide, and an ether-type additive represented by the formula (1):
**R¹-O-R²** **(1)**
[wherein R¹ is a hydrocarbon group having 8 - 24 carbon atoms, and
R² is a group represented by the formula (2): 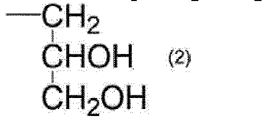 or a group represented by the formula (3):
**-(CH₂CH₂O)ₘH** **(3)**
wherein m is an integer of 1 - 18],
wherein the additive is liquid at 20°C,
further comprising lactic acid, and
further comprising at least one kind of WT1 protein-derived cancer antigen peptide degradation inhibitor selected from the group consisting of decanoic acid, sodium deoxycholate or N-lauroylsarcosine.

2. The transdermal preparation according to claim 1, wherein the WT1 protein-derived cancer antigen peptide is a peptide comprising any amino acid sequence selected from the following amino acid sequences:
Arg-Met-Phe-Pro-Asn-Ala-Pro-Tyr-Leu (SEQ ID NO: 2),
Ser-Leu-Gly-Glu-Gln-Gln-Tyr-Ser-Val (SEQ ID NO: 3),
Cys-Tyr-Thr-Trp-Asn-Gln-Met-Asn-Leu (SEQ ID NO: 4), and
Ala-Leu-Leu-Pro-Ala-Val-Pro-Ser-Leu (SEQ ID NO: 5) or
a peptide comprising an altered amino acid sequence, which is any amino acid sequence selected from SEQ ID NOs: 2, 3, 4 and 5 but containing alteration of amino acid residue(s), and having a CTL induction activity.

3. The transdermal preparation according to claim 1 or 2, wherein the WT1 protein-derived cancer antigen peptide is a peptide shown in the amino acid sequence Arg-Met-Phe-Pro-Asn-Ala-Pro-Tyr-Leu (SEQ ID NO: 2).

4. The transdermal preparation according to any one of claims 1 - 3, wherein the ether-type additive is represented by the formula (4): 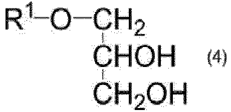 [wherein R¹ is a hydrocarbon group having 8 - 24 carbon atoms,
or the formula (5):
**R¹-O-(CH₂CH₂O)ₙH** **(5)**
wherein R¹ is a hydrocarbon group having 8 - 24 carbon atoms, and
n is an integer of 2 - 18].

5. The transdermal preparation according to any one of claims 1 - 4, wherein the ether-type additive is represented by the formula (6): 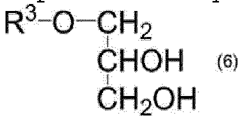 [wherein R³ is a hydrocarbon group having 16 - 18 carbon atoms].

6. The transdermal preparation according to any one of claims 1 - 4, wherein the ether-type additive is represented by the formula (7):
**R¹-O-(CH₂CH₂O)ₚH** **(7)**
[wherein R¹ is a hydrocarbon group having 8 - 24 carbon atoms, and
p is an integer of 2 - 12].

7. The transdermal preparation according to claim 6, wherein R¹ is a branched alkyl group having 8 - 24 carbon atoms, a linear alkenyl group having 8 - 24 carbon atoms or a mixed alkyl group having 8 - 24 carbon atoms.

8. The transdermal preparation according to any one of claims 1 - 4, wherein the ether-type additive is α-monoisostearyl glyceryl ether, monooleyl glyceryl ether, polyoxyethylene isostearyl ether which is liquid at 20°C, polyoxyethylene oleyl ether which is liquid at 20°C, polyoxyethylene alkyl (12 - 14) ether which is liquid at 20°C or a mixture of these.

9. The transdermal preparation according to claim 8, wherein the ether-type additive is α-monoisostearyl glyceryl ether and/or monooleyl glyceryl ether.

10. The transdermal preparation according to any one of claims 1 - 9, which has a dosage form of patches preparation, ointments, gels, creams, lotions or microneedle preparation.

11. The transdermal preparation according to claim 8, which is a patches preparation comprising a support and an adhesive layer formed on one surface of the support, wherein the adhesive layer comprises (1) WT1 protein-derived cancer antigen peptide, (2) at least one ether-type additive selected from the group consisting of α-monoisostearyl glyceryl ether, monooleyl glyceryl ether, polyoxyethylene isostearyl ether which is liquid at 20°C, polyoxyethylene oleyl ether which is liquid at 20°C and polyoxyethylene alkyl (12 - 14) ether which is liquid at 20°C, and (3) an adhesive.

12. A CTL inducer comprising a WT1 protein-derived cancer antigen peptide, and an ether-type additive represented by the formula (1) :
**R¹-O-R²** **(1)**
[wherein R¹ is a hydrocarbon group having 8 - 24 carbon atoms, and
R² is a group represented by the formula (2): 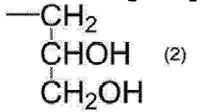 or a group represented by the formula (3):
**-(CH₂CH₂O)ₘH** **(3)**
wherein m is an integer of 1 - 18],
wherein the additive is liquid at 20°C,
further comprising lactic acid, and
further comprising at least one kind of WT1 protein-derived cancer antigen peptide degradation inhibitor selected from the group consisting of decanoic acid, sodium deoxycholate or N-lauroylsarcosine.

## Patentansprüche

1. Transdermales Präparat, umfassend ein von WT1-Protein abgeleitetes Krebs-Antigen-Peptid und ein Additiv des Ethertyps, das durch die Formel (1) dargestellt wird:
**R¹-O-R²** **(1)**
[wobei R¹ eine Kohlenwasserstoffgruppe mit 8 bis 24 Kohlenstoffatomen ist und
R² eine Gruppe, die durch die Formel (2) 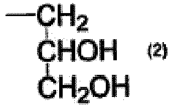 dargestellt wird, oder eine Gruppe, die durch die Formel (3)
**-(CH₂CH₂O)ₘH** **(3)**
dargestellt wird, ist, wobei m eine ganze Zahl von 1 bis 18 ist],
wobei das Additiv bei 20 °C flüssig ist,
weiterhin umfassend Milchsäure und
weiterhin umfassend wenigstens eine Art von Inhibitor des Abbaus des von WT1-Protein abgeleiteten Krebs-Antigen-Peptids, der aus der Gruppe ausgewählt ist, die aus Decansäure, Natriumdesoxycholat oder N-Lauroylsarcosin besteht.

2. Transdermales Präparat gemäß Anspruch 1, wobei das von WT1-Protein abgeleitete Krebs-Antigen-Peptid ein Peptid ist, das eine Aminosäuresequenz umfasst, die aus den folgenden Aminosäuresequenzen ausgewählt ist:
Arg-Met-Phe-Pro-Asn-Ala-Pro-Tyr-Leu (SEQ ID Nr. 2),
Ser-Leu-Gly-Glu-Gln-Gln-Tyr-Ser-Val (SEQ ID Nr. 3),
Cys-Tyr-Thr-Trp-Asn-Gln-Met-Asn-Leu (SEQ ID Nr. 4), und
Ala-Leu-Leu-Pro-Ala-Val-Pro-Ser-Leu (SEQ ID Nr. 5), oder
ein Peptid ist, das eine veränderte Aminosäuresequenz umfasst, bei der es sich um eine Aminosäuresequenz handelt, die aus SEQ ID Nr. 2, 3, 4 und 5 ausgewählt ist, aber eine Veränderung von einem oder mehreren Aminosäureresten enthält und eine CTL-Induktionsaktivität aufweist.

3. Transdermales Präparat gemäß Anspruch 1 oder 2, wobei das von WT1-Protein abgeleitete Krebs-Antigen-Peptid ein Peptid ist, das in der Aminosäuresequenz
Arg-Met-Phe-Pro-Asn-Ala-Pro-Tyr-Leu (SEQ ID Nr. 2)
gezeigt ist.

4. Transdermales Präparat gemäß einem der Ansprüche 1 bis 3, wobei das Additiv des Ethertyps durch die Formel (4): 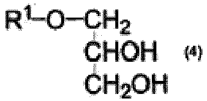 [wobei R¹ eine Kohlenwasserstoffgruppe mit 8 bis 24 Kohlenstoffatomen ist]
oder die Formel (5)
**R¹-O-(CH₂CH₂O)ₙH** **(5)**
[wobei R¹ eine Kohlenwasserstoffgruppe mit 8 bis 24 Kohlenstoffatomen ist und n eine ganze Zahl von 2 bis 18 ist]
dargestellt wird.

5. Transdermales Präparat gemäß einem der Ansprüche 1 bis 4, wobei das Additiv des Ethertyps durch die Formel (6): 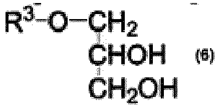 dargestellt wird [wobei R³ eine Kohlenwasserstoffgruppe mit 16 bis 18 Kohlenstoffatomen ist].

6. Transdermales Präparat gemäß einem der Ansprüche 1 bis 4, wobei das Additiv des Ethertyps durch die Formel (7):
**R¹-O-(CH₂CH₂O)ₚH** **(7)**
dargestellt wird [wobei R¹ eine Kohlenwasserstoffgruppe mit 8 bis 24 Kohlenstoffatomen ist und p eine ganze Zahl von 2 bis 12 ist].

7. Transdermales Präparat gemäß Anspruch 6, wobei R¹ eine verzweigte Alkylgruppe mit 8 bis 24 Kohlenstoffatomen, eine lineare Alkenylgruppe mit 8 bis 24 Kohlenstoffatomen oder eine gemischte Alkylgruppe mit 8 bis 24 Kohlenstoffatomen ist.

8. Transdermales Präparat gemäß einem der Ansprüche 1 bis 4, wobei es sich bei dem Additiv des Ethertyps um α-Monoisostearylglycerylether, Monooleylglycerylether, Polyoxyethylenisostearylether, der bei 20 °C flüssig ist, Polyoxyethylenoleylether, der bei 20 °C flüssig ist, Polyoxyethylenalkyl(12-14)ether, der bei 20 °C flüssig ist, oder ein Gemisch von diesen handelt.

9. Transdermales Präparat gemäß Anspruch 8, wobei es sich bei dem Additiv des Ethertyps um α-Monoisostearylglycerylether und/oder Monooleylglycerylether handelt.

10. Transdermales Präparat gemäß einem der Ansprüche 1 bis 9, das in der Darreichungsform Pflasterpräparat, Salben, Gele, Cremes, Lotionen oder Mikronadelpräparat vorliegt.

11. Transdermales Präparat gemäß Anspruch 8, bei dem es sich um ein Pflasterpräparat handelt, das einen Träger und eine auf einer Fläche des Trägers gebildete Kleberschicht umfasst, wobei die Kleberschicht (1) das von WT1-Protein abgeleitete Krebs-Antigen-Peptid, (2) wenigstens ein Additiv des Ethertyps, das aus der Gruppe ausgewählt ist, die aus α-Monoisostearylglycerylether, Monooleylglycerylether, Polyoxyethylenisostearylether, der bei 20 °C flüssig ist, Polyoxyethylenoleylether, der bei 20 °C flüssig ist, und Polyoxyethylen-alkyl(12-14)ether, der bei 20 °C flüssig ist, besteht, und (3) einen Kleber umfasst.

12. CTL-Induktor umfassend ein von WT1-Protein abgeleitetes Krebs-Antigen-Peptid und ein Additiv des Ethertyps, das durch die Formel (1) dargestellt wird:
**R¹-O-R²** **(1)**
[wobei R¹ eine Kohlenwasserstoffgruppe mit 8 bis 24 Kohlenstoffatomen ist und
R² eine Gruppe, die durch die Formel (2) 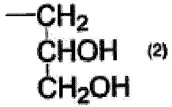 dargestellt wird, oder eine Gruppe, die durch die Formel (3)
**-(CH₂CH₂O)ₘH** **(3)**
dargestellt wird, ist, wobei m eine ganze Zahl von 1 bis 18 ist],
wobei das Additiv bei 20 °C flüssig ist,
weiterhin umfassend Milchsäure und
weiterhin umfassend wenigstens eine Art von Inhibitor des Abbaus des von WT1-Protein abgeleiteten Krebs-Antigen-Peptids, der aus der Gruppe ausgewählt ist, die aus Decansäure, Natriumdesoxycholat oder N-Lauroylsarcosin besteht.

## Revendications

1. Préparation transdermique comprenant un peptide antigénique associé au cancer dérivé de la protéine WT1, et un additif de type éther représenté par la formule (1) :
**R¹-O-R²** **(1)**
[dans laquelle R¹ est un groupe hydrocarboné ayant de 8 à 24 atomes de carbone, et
R² est un groupe représenté par la formule (2) : 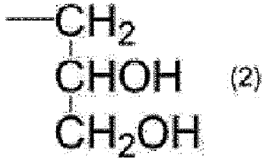 ou un groupe représenté par la formule (3) :
**-(CH₂CH₂O)ₘH** **(3)**
dans laquelle m est un nombre entier de 1 à 18],
dans laquelle l'additif est liquide à 20 °C,
comprenant en outre de l'acide lactique, et
comprenant en outre au moins une sorte d'inhibiteur de dégradation de peptide antigénique associé au cancer dérivé de la protéine WT1 sélectionné dans le groupe constitué de l'acide décanoïque, du désoxycholate de sodium ou de la N-lauroylsarcosine.

2. Préparation transdermique selon la revendication 1, dans laquelle le peptide antigénique associé au cancer dérivé de la protéine WT1 est un peptide comprenant une quelconque séquence d'acides aminés sélectionnée parmi les séquences d'acides aminés suivantes :
Arg-Met-Phe-Pro-Asn-Ala-Pro-Tyr-Leu (SEQ ID NO : 2),
Ser-Leu-Gly-Glu-Gln-Gln-Tyr-Ser-Val (SEQ ID NO : 3),
Cys-Tyr-Thr-Trp-Asn-Gln-Met-Asn-Leu (SEQ ID NO : 4), et
Ala-Leu-Leu-Pro-Ala-Val-Pro-Ser-Leu (SEQ ID NO : 5) ou
un peptide comprenant une séquence d'acides aminés modifiée, qui est une quelconque séquence d'acides aminés sélectionnée parmi SEQ ID NO : 2, 3, 4 et 5 mais contenant une modification d'un ou plusieurs résidus acides aminés, et ayant une activité d'induction des cellules Tc.

3. Préparation transdermique selon la revendication 1 ou 2, dans laquelle le peptide antigénique associé au cancer dérivé de la protéine WT1 est un peptide présenté dans la séquence d'acides aminés Arg-Met-Phe-Pro-Asn-Ala-Pro-Tyr-Leu (SEQ ID NO : 2).

4. Préparation transdermique selon l'une quelconque des revendications 1 à 3, dans laquelle l'additif de type éther est représenté par la formule (4) 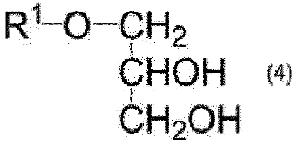 [dans laquelle R¹ est un groupe hydrocarboné ayant de 8 à 24 atomes de carbone,
ou de formule (5) :
**R¹-O-(CH₂CH₂O)ₙH** **(5)**
dans laquelle R¹ est un groupe hydrocarboné ayant de 8 à 24 atomes de carbone, et
n est un nombre entier de 2 à 18].

5. Préparation transdermique selon l'une quelconque des revendications 1 à 4, dans laquelle l'additif de type éther est représenté par la formule (6) : 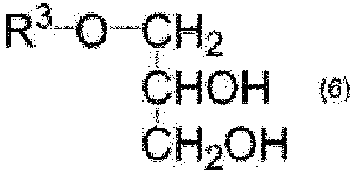 [dans laquelle R³ est un groupe hydrocarboné ayant de 16 à 18 atomes de carbone].

6. Préparation transdermique selon l'une quelconque des revendications 1 à 4, dans laquelle l'additif de type éther est représenté par la formule (7) :
**R¹-O-(CH₂CH₂O)ₚH** **(7)**
[dans laquelle R¹ est un groupe hydrocarboné ayant de 8 à 24 atomes de carbone, et
p est un nombre entier de 2 à 12].

7. Préparation transdermique selon la revendication 6, dans laquelle R¹ est un groupe alkyle ramifié ayant de 8 à 24 atomes de carbone, un groupe alcényle linéaire ayant de 8 à 24 atomes de carbone ou un groupe alkyle mixte ayant de 8 à 24 atomes de carbone.

8. Préparation transdermique selon l'une quelconque des revendications 1 à 4, dans laquelle l'additif de type éther est l'éther glycérylique d'α-monoisostéaryle, l'éther glycérylique de monooléyle, l'éther isostéarylique de polyoxyéthylène qui est liquide à 20 °C, l'éther oléylique de polyoxyéthylène qui est liquide à 20 °C, l'éther alkylique (12-14) de polyoxyéthylène qui est liquide à 20 °C ou un mélange de ceux-ci.

9. Préparation transdermique selon la revendication 8, dans laquelle l'additif de type éther est un éther glycérylique d'α-monoisostéaryle et/ou un éther glycérylique de monooléyle.

10. Préparation transdermique selon l'une quelconque des revendications 1 à 9, dont la forme galénique est une préparation de timbres, des onguents, des gels, des crèmes, des lotions ou une préparation de micro-aiguilles.

11. Préparation transdermique selon la revendication 8, qui est une préparation de timbres comprenant un support et une couche adhésive formée sur une surface du support, dans laquelle la couche adhésive comprend (1) un peptide antigénique associé au cancer dérivé de WT1, (2) au moins un additif de type éther sélectionné dans le groupe constitué de l'éther glycérylique d'α-monoisostéaryle, de l'éther glycérylique de monooléyle, de l'éther isostéarylique de polyoxyéthylène qui est liquide à 20 °C, de l'éther oléylique de polyoxyéthylène qui est liquide à 20 °C, et de l'éther alkylique (12-14) de polyoxyéthylène qui est liquide à 20 °C, et (3) un adhésif.

12. Inducteur des cellules Tc comprenant un peptide antigénique associé au cancer dérivé de la protéine WT1, et un additif de type éther représenté par la formule (1) :
**R¹-O-R²** **(1)**
[dans laquelle R¹ est un groupe hydrocarboné ayant de 8 à 24 atomes de carbone, et
R² est un groupe représenté par la formule (2) : 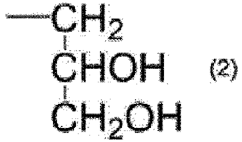 ou un groupe représenté par la formule (3) :
**-(CH₂CH₂O)ₘH** **(3)**
dans laquelle m est un nombre entier de 1 à 18],
dans laquelle l'additif est liquide à 20 °C,
comprenant en outre de l'acide lactique, et
comprenant en outre au moins une sorte d'inhibiteur de dégradation de peptide antigénique associé au cancer dérivé de la protéine WT1 sélectionné dans le groupe constitué de l'acide décanoïque, du désoxycholate de sodium ou de la N-lauroylsarcosine.


### SEQUENCE LISTING

<110> Dainippon Sumitomo Pharma Co., Ltd.
<120> Transdermal agent comprising cancer antigen peptide
<130> 092051
<150> JP 2012-148639
   <151> 2012-07-02
<160> 22
<170> PatentIn version 3.1
<210> 1
   <211> 449
   <212> PRT
   <213> Homo sapiens
<400> 1
   Met Gly Ser Asp Val Arg Asp Leu Asn Ala Leu Leu Pro Ala Val Pro
   1               5                   10                  15
   Ser Leu Gly Gly Gly Gly Gly Cys Ala Leu Pro Val Ser Gly Ala Ala
               20                  25                  30
   Gln Trp Ala Pro Val Leu Asp Phe Ala Pro Pro Gly Ala Ser Ala Tyr
           35                  40                  45
   Gly Ser Leu Gly Gly Pro Ala Pro Pro Pro Ala Pro Pro Pro Pro Pro
       50                  55                  60
   Pro Pro Pro Pro His Ser Phe Ile Lys Gln Glu Pro Ser Trp Gly Gly
   65                  70                  75                  80
   Ala Glu Pro His Glu Glu Gln Cys Leu Ser Ala Phe Thr Val His Phe
                   85                  90                  95
   Ser Gly Gln Phe Thr Gly Thr Ala Gly Ala Cys Arg Tyr Gly Pro Phe
               100                 105                 110
   Gly Pro Pro Pro Pro Ser Gln Ala Ser Ser Gly Gln Ala Arg Met Phe
           115                 120                 125
   Pro Asn Ala Pro Tyr Leu Pro Ser Cys Leu Glu Ser Gln Pro Ala Ile
       130                 135                 140
   Arg Asn Gln Gly Tyr Ser Thr Val Thr Phe Asp Gly Thr Pro Ser Tyr
   145                 150                 155                 160
   Gly His Thr Pro Ser His His Ala Ala Gln Phe Pro Asn His Ser Phe
                   165                 170                 175
   Lys His Glu Asp Pro Met Gly Gln Gln Gly Ser Leu Gly Glu Gln Gln
               180                 185                 190
   Tyr Ser Val Pro Pro Pro Val Tyr Gly Cys His Thr Pro Thr Asp Ser
           195                 200                 205
   Cys Thr Gly Ser Gln Ala Leu Leu Leu Arg Thr Pro Tyr Ser Ser Asp
       210                 215                 220
   Asn Leu Tyr Gln Met Thr Ser Gln Leu Glu Cys Met Thr Trp Asn Gln
   225                 230                 235                 240
   Met Asn Leu Gly Ala Thr Leu Lys Gly Val Ala Ala Gly Ser Ser Ser
                   245                 250                 255
   Ser Val Lys Trp Thr Glu Gly Gln Ser Asn His Ser Thr Gly Tyr Glu
               260                 265                 270
   Ser Asp Asn His Thr Thr Pro Ile Leu Cys Gly Ala Gln Tyr Arg Ile
           275                 280                 285
   His Thr His Gly Val Phe Arg Gly Ile Gln Asp Val Arg Arg Val Pro
       290                 295                 300
   Gly Val Ala Pro Thr Leu Val Arg Ser Ala Ser Glu Thr Ser Glu Lys
   305                 310                 315                 320
   Arg Pro Phe Met Cys Ala Tyr Pro Gly Cys Asn Lys Arg Tyr Phe Lys
                   325                 330                 335
   Leu Ser His Leu Gln Met His Ser Arg Lys His Thr Gly Glu Lys Pro
               340                 345                 350
   Tyr Gln Cys Asp Phe Lys Asp Cys Glu Arg Arg Phe Ser Arg Ser Asp
           355                 360                 365
   Gln Leu Lys Arg His Gln Arg Arg His Thr Gly Val Lys Pro Phe Gln
       370                 375                 380
   Cys Lys Thr Cys Gln Arg Lys Phe Ser Arg Ser Asp His Leu Lys Thr
   385                 390                 395                 400
   His Thr Arg Thr His Thr Gly Lys Thr Ser Glu Lys Pro Phe Ser Cys
                   405                 410                 415
   Arg Trp Pro Ser Cys Gln Lys Lys Phe Ala Arg Ser Asp Glu Leu Val
   420 425 430
   Arg His His Asn Met His Gln Arg Asn Met Thr Lys Leu Gln Leu Ala
   435 440 445
   Leu
<210> 2
   <211> 9
   <212> PRT
   <213> Homo sapiens
<400> 2
   Arg Met Phe Pro Asn Ala Pro Tyr Leu
   1 5
<210> 3
   <211> 9
   <212> PRT
   <213> Homo sapiens
<400> 3
   Ser Leu Gly Glu Gln Gln Tyr Ser Val
   1 5
<210> 4
   <211> 9
   <212> PRT
   <213> Artificial
<220>
   <223> peptide
<400> 4
   Cys Tyr Thr Trp Asn Gln Met Asn Leu
   1 5
<210> 5
   <211> 9
   <212> PRT
   <213> Homo sapiens
<400> 5
   Ala Leu Leu Pro Ala Val Pro Ser Leu
   1 5
<210> 6
   <211> 9
   <212> PRT
   <213> Homo sapiens
<400> 6
   Cys Met Thr Trp Asn Gln Met Asn Leu
   1 5
<210> 7
   <211> 9
   <212> PRT
   <213> Homo sapiens
<400> 7
   Arg Trp Pro Ser Cys Gln Lys Lys Phe
   1 5
<210> 8
   <211> 9
   <212> PRT
   <213> Artificial
<220>
   <223> peptide
<400> 8
   Arg Tyr Phe Pro Asn Ala Pro Tyr Leu
   1 5
<210> 9
   <211> 9
   <212> PRT
   <213> Artificial
<220>
   <223> peptide
<400> 9
   Ala Tyr Leu Pro Ala Val Pro Ser Leu
   1 5
<210> 10
   <211> 9
   <212> PRT
   <213> Homo sapiens
<400> 10
   Asn Gln Met Asn Leu Gly Ala Thr Leu
   1 5
<210> 11
   <211> 9
   <212> PRT
   <213> Artificial
<220>
   <223> peptide
<400> 11
   Asn Tyr Met Asn Leu Gly Ala Thr Leu
   1 5
<210> 12
   <211> 9
   <212> PRT
   <213> Artificial
<220>
   <223> peptide
<400> 12
   Arg Tyr Pro Ser Cys Gln Lys Lys Phe
   1 5
<210> 13
   <211> 9
   <212> PRT
   <213> Artificial
<220>
   <223> peptide
<220>
   <221> MISC_FEATURE
   <222> (1)..(1)
   <223> Xaa is Ser, Ala, Abu, Arg, Lys, Orn, Cit, Leu, Phe or Asn
<400> 13
   Xaa Tyr Thr Trp Asn Gln Met Asn Leu
   1 5
<210> 14
   <211> 9
   <212> PRT
   <213> Artificial
<220>
   <223> peptide
<220>
   <221> MISC_FEATURE
   <222> (1)..(1)
   <223> Xaa is Ser or Ala
<400> 14
   Xaa Met Thr Trp Asn Gln Met Asn Leu
   1 5
<210> 15
   <211> 9
   <212> PRT
   <213> Artificial
<220>
   <223> peptide
<400> 15
   Phe Met Phe Pro Asn Ala Pro Tyr Leu
   1               5
<210> 16
   <211> 9
   <212> PRT
   <213> Artificial
<220>
   <223> peptide
<400> 16
   Arg Leu Phe Pro Asn Ala Pro Tyr Leu
   1               5
<210> 17
   <211> 9
   <212> PRT
   <213> Artificial
<220>
   <223> peptide
<400> 17
   Arg Met Met Pro Asn Ala Pro Tyr Leu
   1               5
<210> 18
   <211> 9
   <212> PRT
   <213> Artificial
<220>
   <223> peptide
<400> 18
   Arg Met Phe Pro Asn Ala Pro Tyr Val
   1               5
<210> 19
   <211> 9
   <212> PRT
   <213> Artificial
<220>
   <223> peptide
<400> 19
   Tyr Met Phe Pro Asn Ala Pro Tyr Leu
   1 5
<210> 20
   <211> 9
   <212> PRT
   <213> Artificial
<220>
   <223> peptide
<400> 20
   Phe Leu Gly Glu Gln Gln Tyr Ser Val
   1 5
<210> 21
   <211> 9
   <212> PRT
   <213> Artificial
<220>
   <223> peptide
<400> 21
   Ser Met Gly Glu Gln Gln Tyr Ser Val
   1 5
<210> 22
   <211> 9
   <212> PRT
   <213> Artificial
<220>
   <223> peptide
<400> 22
   Ser Leu Met Glu Gln Gln Tyr Ser Val
   1 5